# EUROPEAN PATENT SPECIFICATION

(11) **EP 4 502 487 B1**
(45) Date of publication and mention of the grant of the patent: **07.10.2026**
(21) Application number: 23780377.0
(22) Date of filing: 27.03.2023
(51) Int. Cl.: G05B 15/02, F24F 11/89, F24F 11/64, F24F 11/30, F24F 11/46, G01N 33/00, F24F 110/64, F24F 120/10

(54) **SENSOR PLACEMENT DETERMINATION DEVICE AND SENSOR PLACEMENT DETERMINATION METHOD**
SENSORPLATZIERUNGSBESTIMMUNGSVORRICHTUNG UND SENSORPLATZIERUNGSBESTIMMUNGSVERFAHREN
DISPOSITIF DE DÉTERMINATION DE PLACEMENT DE CAPTEUR ET PROCÉDÉ DE DÉTERMINATION DE PLACEMENT DE CAPTEUR

(30) Priority: 31.03.2022 JP 2022061076
(43) Date of publication of application: 05.02.2025
(73) Proprietor: DAIKIN INDUSTRIES, LTD., Osaka-shi, Osaka 530-0001 (JP)
(72) Inventor: SABU, Shunsuke, Osaka-shi, Osaka 530-0001 (JP); SATOU, Daisuke, Osaka-shi, Osaka 530-0001 (JP); KANSHA, Yasuki, Osaka-shi, Osaka 530-0001 (JP)
(74) Representative: Global IP Europe Patentanwaltskanzlei
(86) International application number: PCT/JP2023/012255
(87) International publication number: WO 2023/190367

(56) References cited:
- WO-A1-2021/111617
- WO-A1-2021/235139
- JP-A- 2014 009 498
- JP-A- 2017 532 688

## Description

### TECHNICAL FIELD

The present invention relates to a sensor arrangement determination device and a sensor arrangement determination method.

### BACKGROUND ART

Conventionally, a temperature at a predetermined position has been predicted using a value of an environment sensor installed in a space, and an air conditioner is controlled using the predicted temperature (PTL 1 (Japanese Patent No. 3548114)).

WO 2021/111617 A1 discloses a sensor arrangement determination device that determines arrangement of a sensor which is configured to measure an environment in a space, the space being a room, the environment being a temperature, the sensor arrangement determination device comprising: an acquisition unit configured to acquire second information that is information related to arrangement of the factor in the space, the factor being an air conditioning indoor unit; a determination unit configured to determine an arrangement candidate for the sensor in the space based on the second information; and an output unit configured to output the arrangement candidate determined by the determination unit.

JP 2014-9498 A discloses an insulation performance estimation device that measures an insulation performance of an indoor space in which an air conditioning and heating device is installed, by using a room temperature sensor.

WO 2021/235139 A1 s a control device that estimates CO₂ concentrations at plurality of points in an indoor space 80 by using CO₂ sensors, and control an air conditioning facility 10 based on the estimation result.

### SUMMARY OF INVENTION

### <Technical Problem>

However, when the environment sensor is installed at a position near a heating apparatus, there is a problem in that prediction accuracy decreases when a temperature at a predetermined position is to be predicted, making it impossible to appropriately control the air conditioner.

### <Solution to Problem>

The invention is defined by the system according to independent claim 1 and the method according to the independent claim 8. Optional aspects of the invention are provided by the dependent claims.

A sensor arrangement determination device as a first reference example, which does not fall within the scope of the claimed invention, is a sensor arrangement determination device that determines arrangement of a sensor that measures an environment in a space, and that includes an acquisition unit and a determination unit. The acquisition unit acquires first information and second information. The first information is information in which a factor that influences the environment and an influence of a distance from the factor on the environment are associated with each other. The second information is information related to arrangement of the factor in the space. The determination unit determines an arrangement candidate for the sensor in the space based on the first information and the second information.

With the sensor arrangement determination device, it is possible to take into consideration an influence of a factor on an environment in a space to determine an arrangement candidate for a sensor, making it possible to use the arranged sensor to accurately predict the environment in the space.

A sensor arrangement determination device as a second reference example, which does not fall within the scope of the claimed invention, is the device according to the first reference example, in which the second information includes information related to at least one of a design model, an analysis model, an architectural drawing, and an instrumentation drawing for a building.

With the sensor arrangement determination device, it is possible to acquire space information of a building.

A sensor arrangement determination device as a third reference example, which does not fall within the scope of the claimed invention, is the device according to the first or second reference example, in which the factor that influences the environment is an air conditioner having an indoor unit. The first information includes information related to an influence on the environment in accordance with a distance from the indoor unit, which is determined in accordance with at least one or a combination of any of a model of the air conditioner, a shape of the indoor unit, capability of the air conditioner, an air quantity of conditioned air supplied by the indoor unit, and system information of the air conditioner.

With the sensor arrangement determination device, it is possible to take into consideration an influence of an air conditioner on an environment in a space to determine an arrangement candidate for a sensor.

A sensor arrangement determination device as a fourth reference example, which does not fall within the scope of the claimed invention, is the device according to the third reference example, in which the first information further includes information related to an influence on the environment in accordance with a distance from the indoor unit, which is determined in accordance with at least one or a combination of any of a direction of wind, a blow-out temperature, a blow-out range, and a wind speed of the conditioned air supplied by the indoor unit.

With the sensor arrangement determination device, it is possible to take into consideration an influence of conditioned air supplied by an indoor unit on an environment in a space to determine an arrangement candidate for a sensor.

A sensor arrangement determination device as a fifth reference example, which does not fall within the scope of the claimed invention, is the device according to the first or second reference example, in which the factor that influences the environment is a ventilation device. The first information includes information related to an influence on the environment in accordance with a distance from the ventilation device, which is determined in accordance with at least one or a combination of any of a model of the ventilation device, a shape of the ventilation device, a ventilation quantity of the ventilation device, an air quantity of air supplied by the ventilation device, presence or absence of a total heat exchanger, heat exchange efficiency of the total heat exchanger, and presence or absence of a temperature adjustment function of the ventilation device.

With the sensor arrangement determination device, it is possible to take into consideration an influence of a ventilation device on an environment in a space to determine an arrangement candidate for a sensor in the space.

A sensor arrangement determination device as a sixth reference example, which does not fall within the scope of the claimed invention, is the device according to the first or second reference example, in which the factor that influences the environment is a fixture. The first information includes information related to an influence on the environment in accordance with a distance from the fixture, which is determined in accordance with a type of the fixture.

With the sensor arrangement determination device, it is possible to take into consideration an influence of a fixture on an environment in a space to determine an arrangement candidate for a sensor.

A sensor arrangement determination device as a seventh reference example, which does not fall within the scope of the claimed invention, is the device according to the first or second reference example, in which the factor that influences the environment is a window. The first information is an influence on the environment in accordance with a distance from the window, which is determined in accordance with at least one or a combination of any of a size, a heat transmission coefficient, and a solar radiation acquisition coefficient of the window, and presence or absence of a shield.

With the sensor arrangement determination device, it is possible to take into consideration an influence of a window on an environment in a space to determine an arrangement candidate for a sensor.

A sensor arrangement determination device as an eighth reference example, which does not fall within the scope of the claimed invention, is the device according to the first or second reference example, in which the factor that influences the environment is a door. The first information is an influence on the environment in accordance with a distance from the door, which is determined in accordance with at least one or a combination of any of a size, heat insulation performance, and an opening-and-closing frequency of the door.

With the sensor arrangement determination device, it is possible to take into consideration an influence of a door on an environment in a space to determine an arrangement candidate for a sensor.

A sensor arrangement determination device as a ninth reference example, which does not fall within the scope of the claimed invention, is the device according to the first or second reference example, in which the determination unit calculates a volume or a floor area of the space from the second information, and determines a number of the sensors to be arranged in the space in accordance with the volume or the floor area of the space.

With the sensor arrangement determination device, it is possible to take into consideration a size of a space to determine a numerical value of a sensor, making it possible to avoid arrangement of a large number of sensors and to reduce a cost.

A sensor arrangement determination device as a tenth reference example, which does not fall within the scope of the claimed invention, is the device according to the third or fourth reference example, in which the first information further includes information related to an influence on the environment in accordance with a distance from the indoor unit, which is determined in accordance with a number of the indoor units or a number of refrigerant systems. The determination unit determines a number of the sensors to be arranged in the space in accordance with the number of the indoor units or the number of the refrigerant systems.

With the sensor arrangement determination device, it is possible to take into consideration air conditioner information to determine a numerical value of a sensor, making it possible to determine a number of the sensors suitable for a space in which an indoor unit is installed.

A sensor arrangement determination device according to as an eleventh reference example, which does not fall within the scope of the claimed invention, is the device according to any of the first to ninth reference examples, in which the determination unit determines a number of the sensors to be arranged in the space in accordance with a number of the factors that influence the environment.

With the sensor arrangement determination device, it is possible to take into consideration a number of factors that influence a space to determine a numerical value of a sensor, making it possible to determine an optimal numerical value of the sensor regardless of whether or not a number of factors in the space is large or small.

A sensor arrangement determination device as a twelfth reference example, which does not fall within the scope of the claimed invention, is the device according to any of the first to eighth reference examples, further including an output unit. The output unit outputs an arrangement candidate for the sensor in the space, which is determined by the determination unit.

With the sensor arrangement determination device, it is possible to allow the output unit to output an arrangement candidate for a sensor, making it possible to allow a user to easily acquire the arrangement candidate for the sensor.

A sensor arrangement determination device as a thirteenth reference example, which does not fall within the scope of the claimed invention, is the device according to the twelfth reference example, in which the sensor includes a plurality of sensors arranged in the space for different purposes. The output unit distinguishes each of the plurality of sensors for different purposes from each other, and outputs arrangement candidates for the sensors in the space.

With the sensor arrangement determination device, it is possible to acquire an arrangement candidate for a sensor for each purpose.

A sensor arrangement determination device as a fourteenth reference example, which does not fall within the scope of the claimed invention, is the device according to the twelfth or thirteenth reference example, in which the determination unit determines, for each purpose of arranging the sensors, either positions where there is an influence of a distance from the factor or positions where there is no influence of the distance from the factor as arrangement candidates for the sensors in the space. The output unit outputs the arrangement candidates for the sensors in the space or inappropriate arrangement positions for the sensors in the space for each purpose of arranging the sensors.

With the sensor arrangement determination device, it is possible to acquire inappropriate arrangement positions for sensors, making it possible to take into consideration the inappropriate arrangement positions to determine arrangement of sensors.

A sensor arrangement determination device as a fifteenth reference example, which does not fall within the scope of the claimed invention, is the device according to the thirteenth or fourteenth reference example, in which the sensors for different purposes include: a first sensor; and a second sensor. The first sensor is constantly installed in the space. The second sensor is to be retrieved after data related to the environment in the space has been acquired. The output unit outputs a notification of a timing at which the second sensor is to be retrieved from the space or a notification of a timing at which the second sensor ends acquisition of the data.

With the sensor arrangement determination device, it is possible to acquire a timing at which a sensor is to be retrieved or a timing at which the sensor ends acquisition of data.

A sensor arrangement determination method as a sixteenth reference example, which does not fall within the scope of the claimed invention, is a sensor arrangement determination method of determining arrangement of a sensor that measures an environment in a space, and that includes: an acquisition step; and a determination step. At the acquisition step, first information and second information are acquired. The first information is information in which a factor that influences the environment and an influence of a distance from the factor on the environment are associated with each other. The second information is information related to arrangement of the factor in the space. At the determination step, an arrangement candidate for the sensor in the space is determined based on the first information and the second information.

With the sensor arrangement determination method, it is possible to take into consideration an influence of a factor in a space on an environment to determine an arrangement candidate for a sensor, making it possible to accurately predict the environment in the space.

A sensor arrangement determination device as a seventeenth reference example, which does not fall within the scope of the claimed invention, includes an analysis unit and a determination unit. The analysis unit performs, in relation to an influence of a factor that influences an environment in a space, analysis of a hot air flow in the space. The determination unit determines an arrangement candidate for a sensor in the space based on a result of the analysis of the hot air flow in the space.

With the sensor arrangement determination device, it is possible to take into consideration a result of analysis of a hot air flow in a space to determine an arrangement candidate for a sensor, making it possible to accurately predict an environment in the space.

### BRIEF DESCRIPTION OF DRAWINGS

[Fig. 1] Fig. 1 is a functional block diagram of an air-conditioning control system.
[Fig. 2] Fig. 2 is a diagram illustrating an example of a target space.
[Fig. 3] Fig. 3 is a diagram illustrating an example of arrangement of air conditioners in a target space.
[Fig. 4] Fig. 4 is a flowchart for the air-conditioning control system.
[Fig. 5] Fig. 5 is a diagram for explaining an example of air conditioner information and space information.
[Fig. 6] Fig. 6 is a diagram for explaining an example of an inappropriate arrangement location for a constant sensor.
[Fig. 7] Fig. 7 is a diagram for explaining an example of installation ranges for sensors.
[Fig. 8] Fig. 8 is a diagram illustrating another example of the target space.
[Fig. 9A] Fig. 9A is a diagram for explaining another example of inappropriate arrangement locations for a constant sensor.
[Fig. 9B] Fig. 9B is a diagram for explaining still another example of inappropriate arrangement locations for a constant sensor.
[Fig. 10] Fig. 10 is a functional block diagram of an air-conditioning control system according to a modification example.
[Fig. 11] Fig. 11 is a diagram illustrating another example of installation ranges for sensors.
[Fig. 12] Fig. 12 is a diagram for explaining an installation number of constant sensors.
[Fig. 13] Fig. 13 is a functional block of an air-conditioning control system according to a modification example.

### DESCRIPTION OF EMBODIMENTS

### (1) Overview of Air-conditioning Control System

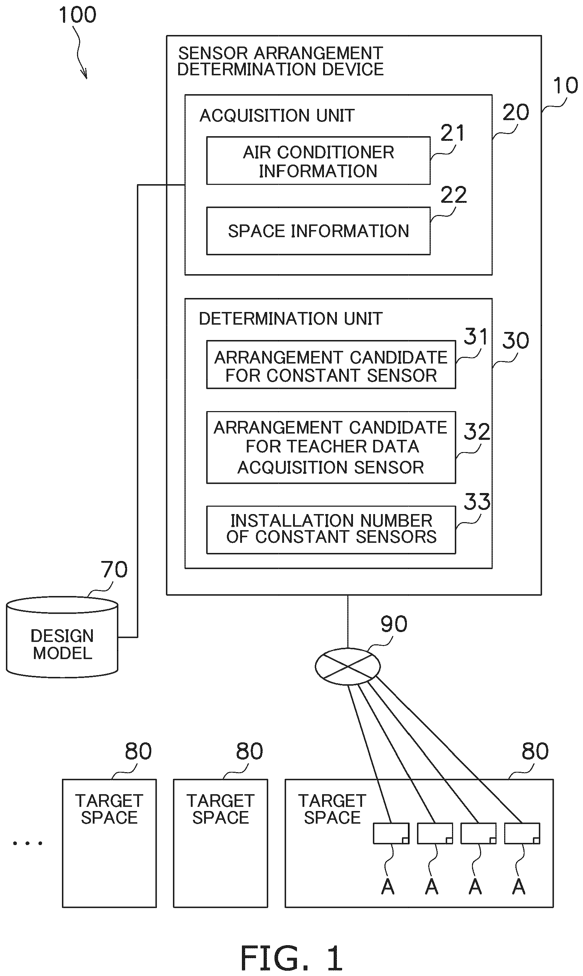

An air-conditioning control system 100 according to the present embodiment is a system provided to perform air-conditioning control in a room that is a target space 80. As illustrated in Fig. 1, the air-conditioning control system 100 includes an air conditioner A and a sensor arrangement determination device 10.

The air-conditioning control system 100 according to the present embodiment corresponds to the sensor arrangement determination device 10. The sensor arrangement determination device 10 is a device that implements a sensor arrangement candidate determination method described in the claims.

The sensor arrangement determination device 10 communicates with a device related to air conditioning in the target space 80 via a network 90. In the present embodiment, the device related to air conditioning in the target space 80 is the air conditioner A. The air conditioner A is an air conditioning indoor unit (an indoor unit) installed in the space.

The sensor arrangement determination device 10 determines an arrangement candidate for a sensor in the target space 80. The sensor includes a constant sensor S1 and a teacher data acquisition sensor S2.

### (2) Configuration of Air-conditioning Control System

### (2-1) Sensor Arrangement Determination Device

The sensor arrangement determination device 10 is achieved by a computer. As illustrated in Fig. 1, the sensor arrangement determination device 10 includes an acquisition unit 20 and a determination unit 30. The computer 10 includes a control computation device and a storage device. As the control computation device, it is possible to use a processor such as a central processing unit (CPU) or a graphics processing unit (GPU). The control computation device reads a program stored in the storage device and performs predetermined image processing and computation processing in accordance with the program. Furthermore, the control computation device is able to write a result of computation in the storage device and read information stored in the storage device in accordance with the program. The acquisition unit 20 and the determination unit 30 illustrated in Fig. 1 are functional blocks that vary in type and that are achieved by the control computation device. These functional blocks appear as the control computation device executes the program.

### (2-1-1) Acquisition Unit

The acquisition unit 20 acquires air conditioner information (first information) 21 that is information in which a factor that influences an environment in the target space 80 and an influence of a distance from the factor on the environment in the target space 80 are associated with each other. In the present embodiment, the environment in the target space 80 is a temperature. Furthermore, in the present embodiment, a factor that influences the temperature is the air conditioner (indoor unit) A.

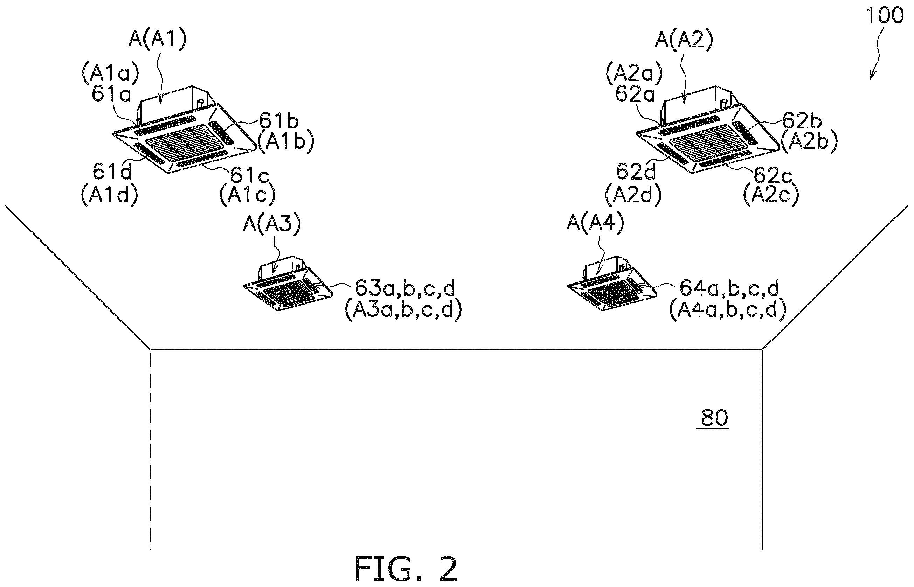

As illustrated in Fig. 2, the air-conditioning control system 100 according to the present embodiment includes a plurality of air conditioners A1 to A4 in the target space 80.

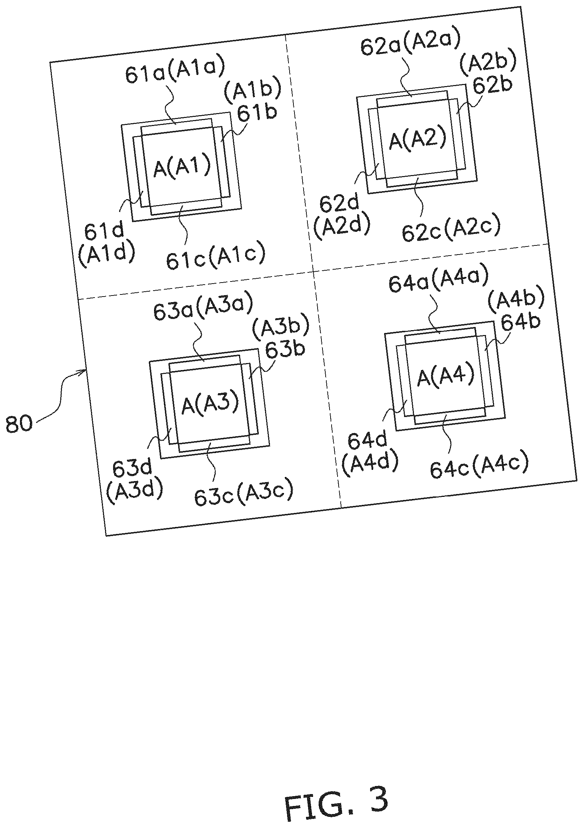

As illustrated in Fig. 3, the plurality of air conditioners A1 to A4 are provided with blow-out ports A1a to A4d, each four along four sides. Then, the air conditioners A1 to A4 have flaps 61a to 64d for adjusting directions of wind that is blown out of the blow-out ports A1a to A4d.

In the present embodiment, the air conditioner information 21 is information in which the air conditioner A and an influence of a distance from the air conditioner A are associated with each other. When the distance from the air conditioner A is short, the influence of the air conditioner A on the temperature in the target space 80 becomes large. For example, although, when a linear distance from the air conditioner A is approximately 3 m or shorter, the influence of the air conditioner A on the temperature in the target space 80 becomes large, the linear distance from the air conditioner A is not limited to this example.

The air conditioner information 21 includes information related to the influence on the temperature in accordance with the distance from the air conditioner A, which is determined in accordance with at least one or a combination of any of a model of the air conditioner A, a shape of the indoor unit (air conditioner) A, capability of the air conditioner A, an air quantity of conditioned air supplied by the air conditioner A, and refrigerant system information (system information) of the air conditioner A.

Examples of the model of the air conditioner A include a room air conditioner for a house, an air conditioner for a store or an office, and multi-air conditioners for a building. Furthermore, examples of the shape of the indoor unit (air conditioner) A include a ceiling-embedded cassette type, a ceiling-suspended type, and a floor-mounted type. **In** the present embodiment, the shape of the indoor unit A is a ceiling-embedded cassette type.

When the capability of the air conditioner A is large, the influence of the air conditioner A on the temperature in the target space 80 becomes large. For example, although, when the capability of the air conditioner A is approximately 2.8 kW or greater, the influence of the air conditioner A on the temperature in the target space 80 becomes large, the capability of the air conditioner A is not limited to this example.

When the air quantity of conditioned air supplied by the air conditioner A is large, the influence of the air conditioner A on the temperature in the target space 80 becomes large. For example, although, when the air quantity of conditioned air supplied by the air conditioner A is approximately 300 m3/min or larger, the influence of the air conditioner A on the temperature in the target space 80 becomes large, the air quantity of conditioned air supplied by the air conditioner A is not limited to this example.

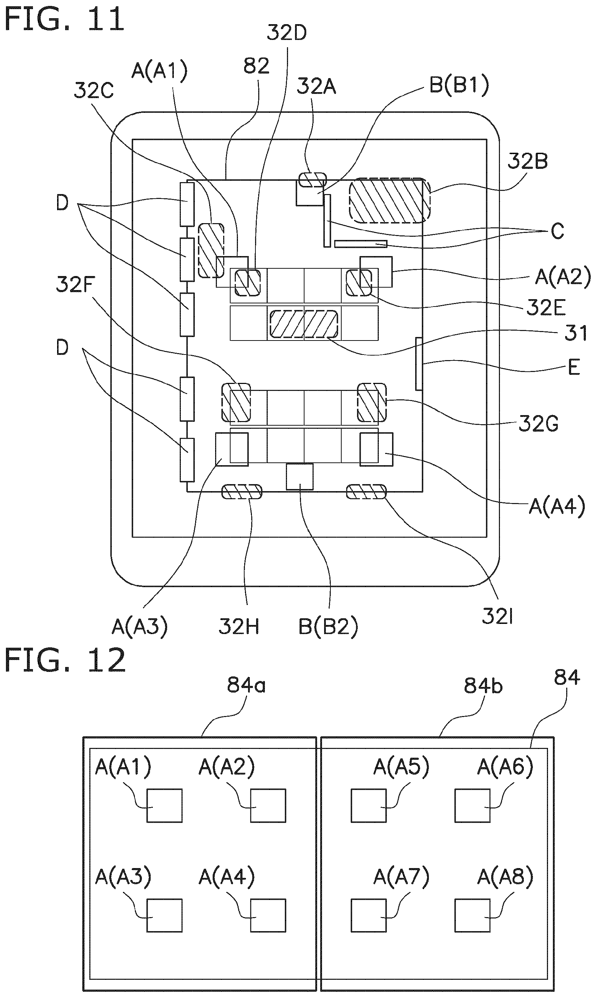

The refrigerant system information of the air conditioners A is information related to coupling between the indoor unit and an outdoor unit. A plurality of indoor units coupled to one outdoor unit via refrigerant pipes are assumed to belong to a single refrigerant system. For example, it is assumed that, in a first refrigerant system, the plurality of indoor units A1 to A4 be coupled to a first outdoor unit via refrigerant pipes, and, in a second refrigerant system, a plurality of indoor units A5 to A8 (see Fig. 12) are coupled to a second outdoor unit via refrigerant pipes.

The refrigerant system information of the air conditioners A includes information related to a fact that which refrigerant system each indoor unit belongs to, among the plurality of refrigerant systems. For example, the refrigerant system information of the air conditioners A includes information about a fact that the indoor units A1 to A4 belong to the first refrigerant system.

Furthermore, the air conditioner information 21 may further include information related to the influence on the temperature in accordance with the distance from the air conditioner A, which is determined in accordance with at least one or a combination of any of a direction of wind, a blow-out temperature, a blow-out range, and a wind speed of conditioned air supplied by the air conditioner A.

As illustrated in Figs. 2 and 3, the flaps 61a to 64d of the air conditioners (indoor units) A1 to A4 are provided, respectively, inside the four sides on an outer periphery of a lower surface of each one of the indoor units A1 to A4, at positions corresponding to the blow-out ports A1a to A4d to extend in parallel with the sides, respectively. A control unit (not illustrated) performs control to change an angle of each of the flaps 61a to 64d of each of the indoor units A with respect to each of the blow-out ports A1a to A4d to make it possible to direct a blow-out airflow toward a predetermined position in the target space 80.

A fan (not illustrated) is provided in each of the indoor units A1 to A4, allowing the control unit to adjust a speed of the blow-out airflow that is sent out of each of the blow-out ports A1a to A4d.

The influence of the air conditioner A on the temperature in the target space 80 becomes large in a direction of blown-out wind of conditioned air supplied by the air conditioner A. In the present embodiment, the air conditioners A1 to A4 blow out conditioned air in four directions from the blow-out ports A1a to A4d, respectively. The air conditioners A1 to A4 may blow out conditioned air in, for example, any two directions, among the directions, from the blow-out ports A1a to A4d, respectively. Among the blow-out ports A1a to A4d of the air conditioners A1 to A4, the influence of the air conditioner A on the temperature in the target space 80 becomes large in a direction of blown-out conditioned air.

Furthermore, when a temperature difference between a blow-out temperature of the air conditioner A and an indoor temperature in the target space 80 is large, the influence of the air conditioner A on the temperature in the target space 80 becomes large due to the blow-out temperature of the air conditioner A. For example, although, when a temperature difference between the blow-out temperature of the air conditioner A and the indoor temperature in the target space 80 is approximately 10°C or higher, the influence of the air conditioner A on the temperature in the target space 80 becomes large due to the blow-out temperature of the air conditioner A, a temperature difference between the blow-out temperature of the air conditioner A and the indoor temperature in the target space 80 is not limited to this example.

Furthermore, the influence of the air conditioner A on the temperature in the target space 80 becomes large within a blow-out range of the indoor unit A. In the present embodiment, it is possible to allow the indoor units A1 to A4 to operate by changing the blow-out range of each of the indoor units A within a range between 30° and 90°, as viewed from a ceiling, by changing the directions of the flaps 61a to 64d.

When the wind speed of the indoor unit A is high, the influence of the air conditioner A on the temperature in the target space 80 becomes large. For example, although, when the wind speed of each of the indoor units A is approximately 1 m/s or faster at a distance of 0 m from each of the blow-out ports A1a to A4d of the air conditioners A, the influence of the air conditioner A on the temperature in the target space 80 becomes large, the wind speed of the indoor unit A is not limited to this example.

Furthermore, the acquisition unit 20 acquires space information (second information) 22 that is information related to arrangement of a factor that influences the environment in the target space 80. In the present embodiment, the space information 22 is information related to arrangement of the air conditioner A in the target space 80. Furthermore, the space information 22 includes information related to a design model 70 of a building that includes the target space 80. In the present embodiment, the acquisition unit 20 acquires information related to arrangement of the air conditioner A in the target space 80 in the building from the design model 70 of the building. The space information 22 is not limited to the design model 70 of the building, and may include information related to at least one of an analysis model, an architectural drawing, and an instrumentation drawing for the building.

### (2-1-2) Determination Unit

The determination unit 30 determines an arrangement candidate 31 for the constant sensor S1 in the target space 80 and an arrangement candidate 32 for the teacher data acquisition sensor S2 in the target space 80 based on the air conditioner information 21 and the space information 22.

In the present embodiment, the determination unit 30 determines the arrangement candidate 31 for the constant sensor S1 in the target space 80 and the arrangement candidate 32 for the teacher data acquisition sensor S2 in the target space 80 based on the air conditioner information 21 in which the air conditioner A and the influence of the distance from the air conditioner A are associated with each other and the space information 22 related to arrangement of the air conditioner A in the target space 80.

Furthermore, the determination unit 30 calculates a floor area of the target space 80 from the space information 22, and determines a number 33 of the constant sensors S1 to be arranged in the target space 80 in accordance with the floor area of the target space 80. Based on a shape and a size of the target space 80, a number of the air conditioners A, and target accuracy of prediction of a temperature, the determination unit 30 may divide the target space 80 into several areas each having a predetermined floor area, and assume that each one of the constant sensors S1 be installed in each of the areas to determine the number 33 of the constant sensors S1.

In the present embodiment, the determination unit 30 calculates the floor area of the target space 80 from the design model 70, and determines the number 33 of the constant sensors S1 to be arranged in the target space 80 in accordance with the floor area of the target space 80. It is preferable that one constant sensor S1 is arranged per a floor area ranging from 100 m² to 150 m² in the target space 80. In the present embodiment, one constant sensor S1 is arranged per a floor area of 100 m² in the target space 80. For example, when a floor area of the target space 80 is 100 m², the determination unit 30 determines that the number 33 of the constant sensors S1 to be arranged in the target space 80 is "1".

### (3) Sensor

The sensor is an environment sensor that measures the environment in the target space 80. The sensor includes the constant sensor S1 that is constantly installed in the target space 80 and the teacher data acquisition sensor S2 that is installed in the target space 80 when teacher data is to be acquired.

Examples of the environment in the target space 80 include temperature, humidity, and carbon dioxide concentration. Examples of the environment sensor include a temperature sensor, a carbon dioxide sensor, and a humidity sensor. In the present embodiment, the sensor is a temperature sensor.

### (3-1) Constant sensor

The constant sensor S1 is the temperature sensor for acquiring data related to the temperature in the target space 80. In the present embodiment, the constant sensor S1 acquires data related to the temperature in the target space 80.

The constant sensor S1 is constantly installed in the target space 80 before actual implementation and during the implementation of air-conditioning control by the air-conditioning control system 100.

The constant sensor S1 is arranged at a position determined by the sensor arrangement determination device 10 in the air-conditioning control system 100. The constant sensor S1 is arranged at a location where there is a less influence of the air conditioner A on the temperature in the target space 80.

### (3-2) Teacher data acquisition sensor

The teacher data acquisition sensor S2 is a temperature sensor for acquiring teacher data related to the temperature in the target space 80. In the present embodiment, the teacher data acquisition sensor S2 acquires teacher data related to the temperature in the target space 80.

A machine learning device (not illustrated) uses the teacher data to perform learning of a soft-sensing prediction model. The machine learning device performs learning by associating a value of the data of the constant sensor S1, which serves as an explanatory variable of the soft-sensing prediction model, and a value of the teacher data of the teacher data acquisition sensor S2, which serves as an objective variable of the soft-sensing prediction model, with each other. The soft-sensing prediction model uses the value of the data of the constant sensor S1 as the explanatory variable to predict a temperature at a position where the teacher data acquisition sensor S2 has been installed.

The teacher data acquisition sensor S2 is installed at a position (a prediction position) at which the soft-sensing prediction model is used to predict the temperature in the target space 80 before actual implementation of air-conditioning control by the air-conditioning control system 100.

After start of actual implementation of air-conditioning control by the air-conditioning control system 100, the teacher data acquisition sensor S2 is retrieved, and the created soft-sensing prediction model is used to predict a parameter (the temperature) to be applied to visualization of temperature distribution and the air-conditioning control, for example.

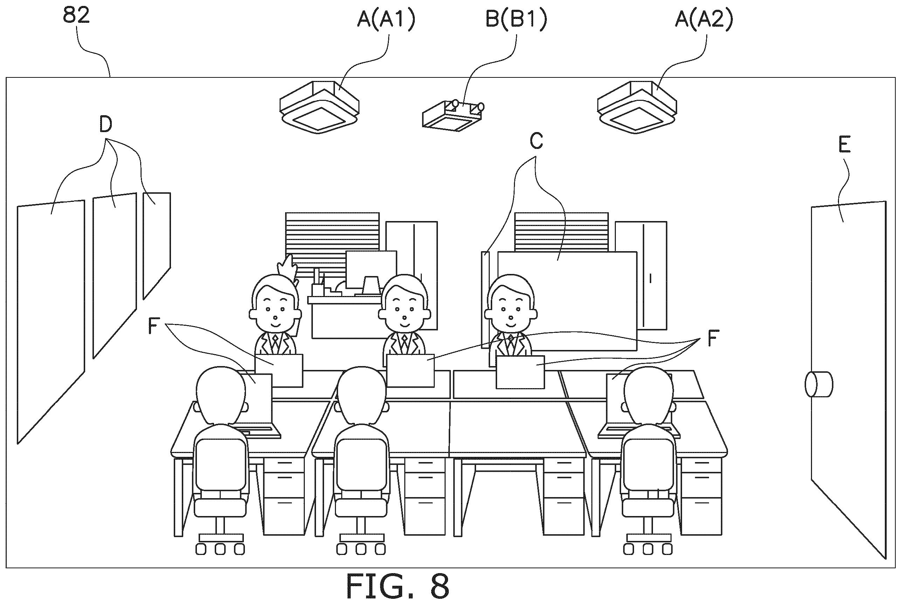

The teacher data acquisition sensor S2 is arranged at a position determined by the sensor arrangement determination device 10 in the air-conditioning control system 100. The teacher data acquisition sensor S2 is arranged, for example, at a position near a disturbance element having a strong influence on the temperature in the target space 80. Example disturbance elements having a strong influence on the temperature in the target space 80 include a ventilation device B and a window D, for example, (see Fig. 8). Alternatively, the teacher data acquisition sensors S2 are uniformly arranged in the target space 80 wholly.

### (4) Overall Operation of Air-conditioning Control System

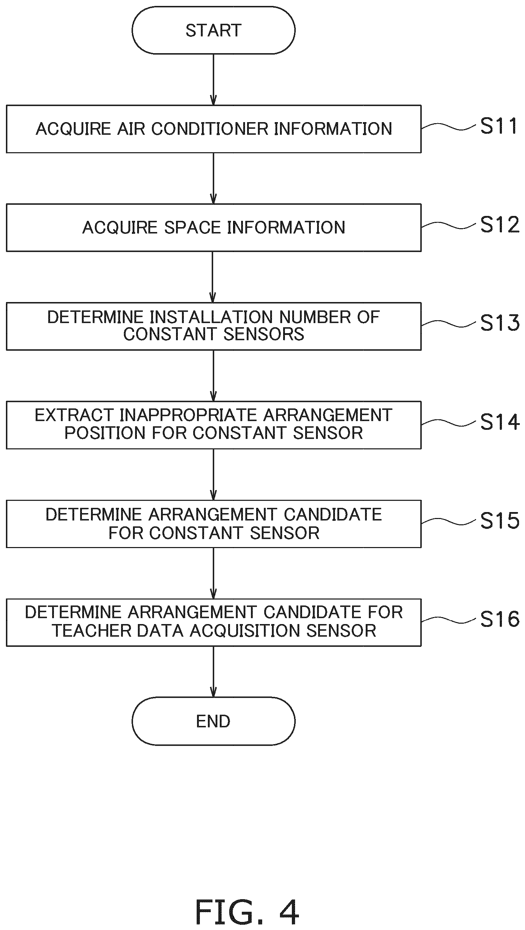

A flowchart for the air-conditioning control system 100 is illustrated in Fig. 4. In Fig. 4, a case where the air conditioners A1 to A4 are arranged in the target space 80 will now be described herein as an example.

At step S11, the acquisition unit 20 acquires the air conditioner information 21. In the present embodiment, the acquisition unit 20 acquires the air conditioner information 21 that is information in which the air conditioners A1 to A4 and influences of distances from the air conditioners A1 to A4 are associated with each other.

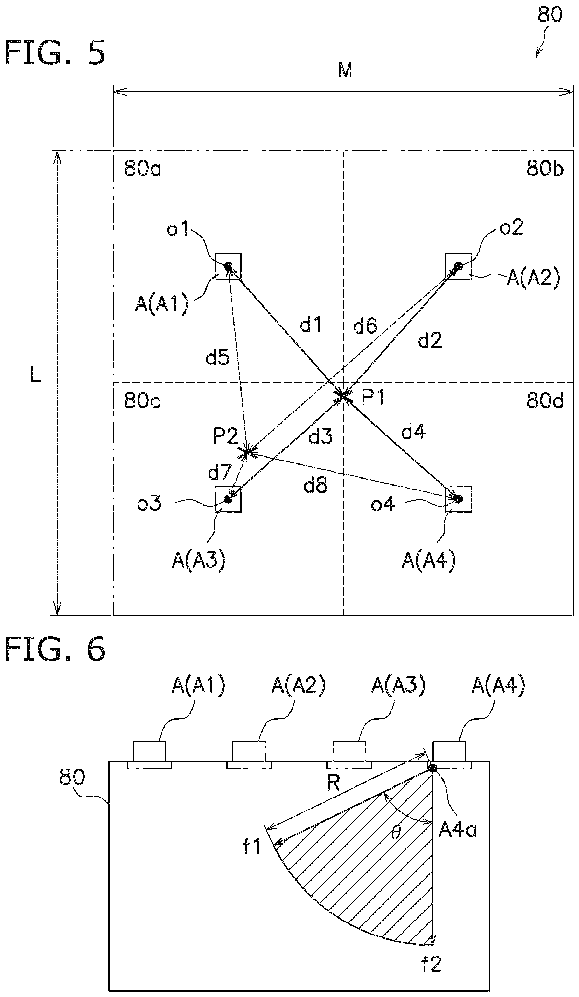

As illustrated in Fig. 5, for example, a distance from a center position o1 of the air conditioner A1 to a predetermined position P1 near a center of the target space 80 is d1. A distance from a center position o2 of the air conditioner A2 to the predetermined position P1 near the center of the target space 80 is d2. A distance from a center position o3 of the air conditioner A3 to the predetermined position P1 near the center of the target space 80 is d3. A distance from a center position o4 of the air conditioner A4 to the predetermined position P1 near the center of the target space 80 is d4.

Furthermore, for example, a distance from the center position o1 of the air conditioner A1 to a predetermined position P2 in a region 80c in the target space 80 is d5. A distance from the center position o2 of the air conditioner A2 to the predetermined position P2 in the region 80c in the target space 80 is d6. A distance from the center position o3 of the air conditioner A3 to the predetermined position P2 in the region 80c in the target space 80 is d7. A distance from the center position o4 of the air conditioner A4 to the predetermined position P2 in the region 80c in the target space 80 is d8.

The acquisition unit 20 acquires, as the air conditioner information 21, for example, information in which the air conditioner A1 and the distance d1 from the center position o1 of the air conditioner A1 to the predetermined position P1 in a region 80a in the target space 80 are associated with each other. Thereafter, processing proceeds to step S12.

At step S12, the acquisition unit 20 acquires the space information 22. In the present embodiment, the second information 22 that is information related to arrangement of the air conditioners A1 to A4 in the target space 80 in the building is acquired from the design drawing 70.

As illustrated in Fig. 5, the target space 80 includes the region 80a, a region 80b, the region 80c, and a region 80d. The air conditioner A1 is arranged in the region 80a in the target space 80. The air conditioner A2 is arranged in the region 80b in the target space 80. The air conditioner A3 is arranged in the region 80c in the target space 80. The air conditioner A4 is arranged in the region 80d in the target space 80. Thereafter, the processing proceeds to step S13.

At step S13, the determination unit 30 determines the installation number 33 of the constant sensors S1. At step S13, the target space 80 is divided into predetermined areas based on the air conditioner information 21 acquired at step S11 and the space information 22 acquired at step S12, and it is assumed that each one of the constant sensors S1 be installed in each of the areas to determine the installation number 33 of the constant sensors S1.

In the present embodiment, the determination unit 30 calculates the floor area of the target space 80 from the space information 22 acquired at step S12, and determines the number 33 of the constant sensors S1 to be arranged in the target space 80 in accordance with the floor area of the target space 80.

As illustrated in Fig. 5, the floor area of the target space 80 has a square shape having an area of 100 m² and having a side L extending at a length of 10 m and a side M extending at a length of 10 m. Therefore, the determination unit 30 determines that the installation number 31 of the constant sensors S1 in the target space 80 is "1". Thereafter, the processing proceeds to step S14.

At step S14, the determination unit 30 extracts an inappropriate arrangement position for the constant sensor S1. **In** the present embodiment, the inappropriate arrangement position for the constant sensor S1 lies within a reaching range of a blow-out airflow from the air conditioner A.

The determination unit 30 estimates, from the shape of the air conditioner (indoor unit) A, the capability of the air conditioner A, and the air quantity of the air conditioner A, a maximum air quantity of the air conditioner A and the reaching range of the blow-out airflow in each direction of wind, and extracts the reaching range of the blow-out airflow from the air conditioner A as the inappropriate arrangement position for the constant sensor S1. Furthermore, the reaching range of the blow-out airflow from the air conditioner A may be estimated based on a physical distance from the air conditioner A.

By associating characteristics of the model of the indoor unit A and the reaching range of the blow-out airflow with each other, and by acquiring the air conditioner information 21, it is possible to acquire the reaching range of the blow-out airflow from the air conditioner A in the target space 80. Although the reaching range of the blow-out airflow from the air conditioner A ranges from approximately 0 m to approximately 3 m from each of the blow-out ports of the air conditioner A, the reaching range of the blow-out airflow from the air conditioner A is not limited to this example.

Fig. 6 is a diagram for explaining an example of the inappropriate arrangement location for the constant sensor S1. In the present embodiment, as illustrated in Fig. 6, the reaching range of the blow-out airflow from the blow-out port A4a of the air conditioner A4 is, for example, a range where a distance R from the blow-out port A4a of the air conditioner A4 is 3 m, and a direction f1 in which the air conditioner A4 and the ceiling in the target space 80 form an angle of 30° and a direction f2 in which the air conditioner A4 and the ceiling in the target space 80 form an angle of 90° form an angle of θ. Thereafter, the processing proceeds to step S15.

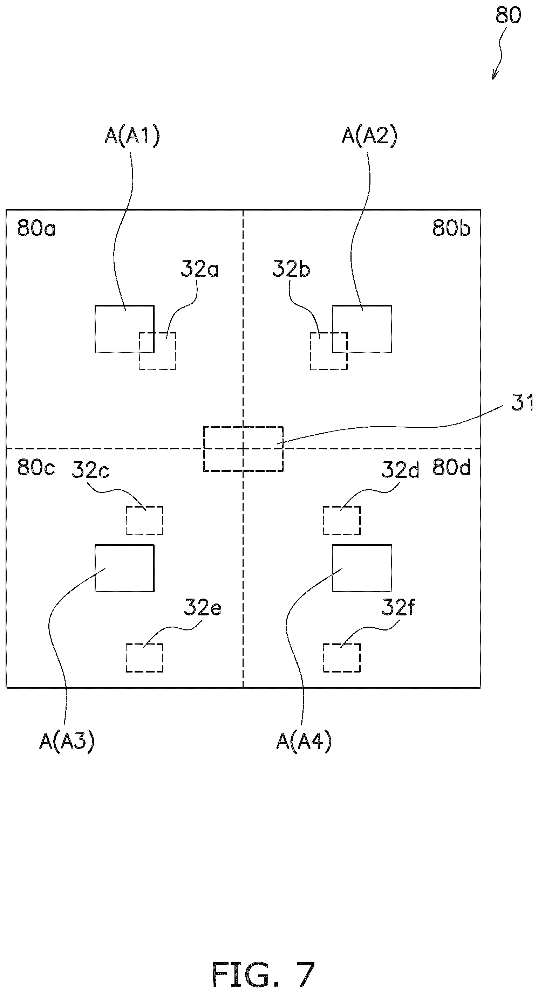

At step S15, the determination unit 30 determines the arrangement candidate 31 that is a recommended installation position (range) for the constant sensor S1. At step S15, the inappropriate arrangement position for the constant sensor S1, which is extracted at step S14, is excluded from the target space 80. In the present embodiment, the reaching range of the blow-out airflow from the air conditioner A is excluded from the target space 80. Furthermore, an intermediate position from the air conditioners A belonging to the areas divided at step S13, in a region acquired by excluding the reaching range of the blow-out airflow from the air conditioner A from the target space 80, is set as a recommended installation position (range) for the constant sensor S1. In the present embodiment, an intermediate position from the indoor units A1 to A4 provided in the regions 80a to 80d in the target space 80 is determined as the arrangement candidate 31 (see Fig. 7) for the constant sensor S1. Thereafter, the processing proceeds to step S16.

At step S16, the determination unit 30 determines the arrangement candidate 32 that is a recommended installation position (range) for the teacher data acquisition sensor S2. At step S16, the determination unit 30 extracts positions 32a, 32b, 32c, and 32d (see Fig. 7) at which the teacher data acquisition sensors S2 should be installed, based on the range in which the inappropriate arrangement position for the constant sensor S1 lies, which is extracted at step S14. The positions 32a, 32b, 32c, and 32d at which the teacher data acquisition sensors S2 should be installed lie within the reaching ranges of the blow-out airflows from the air conditioners A1 to A4.

Based on the air conditioner information 21 acquired at step S11 and the space information 22 acquired at step S12, the determination unit 30 may determine positions at which there are strong influences of the air conditioners A1 to A4 on the temperature through determination using a threshold value, for example, and may set the positions as the positions 32a, 32b, 32c, and 32d at which the teacher data acquisition sensors S2 should be installed.

Then, the determination unit 30 acquires a plurality of other positions 32e and 32f (see Fig. 7) to allow the teacher data acquisition sensors S2 to be evenly scattered in the target space 80.

The determination unit 30 determines the positions 32a, 32b, 32c, and 32d at which the teacher data acquisition sensors S2 should be installed and the plurality of other positions 32e and 32f for the teacher data acquisition sensors S2 as the arrangement candidates 32 for the teacher data acquisition sensors S2.

Fig. 7 is a diagram for explaining an example of installation ranges for sensors in the target space 80. It is assumed that one constant sensor S1 and six teacher data acquisition sensors S2 (S2a to S2f) be arranged in the target space 80. As illustrated in Fig. 7, the target space 80 includes the regions 80a to 80d. The air conditioner A1 is arranged in the region 80a in the target space 80. The air conditioner A2 is arranged in the region 80b in the target space 80. The air conditioner A3 is arranged in the region 80c in the target space 80. The air conditioner A4 is arranged in the region 80d in the target space 80.

The constant sensor S1 is arranged at the arrangement candidate 31 that is the intermediate position from the air conditioners A1 and A4 in the target space 80. The teacher data acquisition sensor S2a is arranged at the arrangement candidate 32a near the air conditioner A1. The teacher data acquisition sensor S2b is arranged at the arrangement candidate 32b near the air conditioner A2. The teacher data acquisition sensor S2c is arranged at the arrangement candidate 32c near the air conditioner A3. The teacher data acquisition sensor S2d is arranged at the arrangement candidate 32d near the air conditioner A4. The teacher data acquisition sensor S2e is arranged at the arrangement candidate 32e that is a position at which no airflows from the air conditioners A1 to A4 are received. The teacher data acquisition sensor S2f is arranged at the arrangement candidate 32f that is a position at which no airflows from the air conditioners A1 to A4 are received.

### (5) Features

### (5-1)

The sensor arrangement determination device 10 according to the present embodiment is a sensor arrangement determination device 10 that determines arrangement of a sensor that measures the temperature in the target space 80, and that includes the acquisition unit 20 and the determination unit 30. The acquisition unit 20 acquires the air conditioner information 21 and the space information 22. The air conditioner information 21 is information in which the air conditioner A that influences the temperature and the influence of the distance from the air conditioner A on the temperature are associated with each other. The space information 22 is information related to arrangement of the air conditioner A in the target space 80. The determination unit 30 determines the arrangement candidate 31 for the constant sensor S1 and the arrangement candidate 32 for the teacher data acquisition sensor S2 in the target space 80 based on the air conditioner information 21 and the space information 22.

In a conventional air-conditioning control system, a value of a sensor arranged in an indoor unit or a remote controller is used to control an air conditioner. Therefore, since a situation of a temperature and an airflow in a spatial residential area (approximately 1.7 m high from a floor) is not taken into consideration, there has been a demand for air conditioning due to temperature unevenness and a draft (wind hitting) feeling.

For example, in actual temperature distribution in an air conditioned space, an aspect of temperature change differs depending on a position in the air conditioned space, such as a position at which whether or not a blow-out airflow from an air conditioner reaches, a position at a different height in the air conditioned space, a position at which there is a strong influence or a weak influence of disturbance, and a position where there are influences of indoor units in a plurality of refrigerant systems. Since, conventionally, a local value is acquired and reflected on air-conditioning control when controlling an air conditioner using a sensor installed in a space, it may become impossible to construct a target environment when the sensor is installed at a position where there is a singular point, such as a location near a heating apparatus, an area where many people are crowded, a location behind a partition, and a position where there is a strong influence of solar radiation.

Furthermore, not only in controlling an air conditioner, but also in acquiring parameter distribution in a space for aiming at visualization of parameter distribution in an air conditioned space, a position of an environment sensor in the space is important. Example parameters in an air conditioned space include temperature, humidity, CO₂, airflow, and degree of comfort (predicted mean vote or PMV). By measuring parameter distribution in a residential area in a space to perform air-conditioning control and ventilation control or visualization of the parameter distribution in accordance with how an actual person feels, it is possible to assist changing of settings of an air conditioner, to allow a person in a room to select a comfortable place for himself/herself, and to assist examination of introduction of equipment.

When an object is visualization of parameter distribution, it may not be possible to correctly acquire the parameter distribution in a whole space, unless not only an environment occupying majority of the space, but also a portion that influences parameters and various types of singular points occurring in the space are measured.

Furthermore, when parameter distribution is to be predicted, a constant sensor constantly installed in a space and a teacher data acquisition sensor that is to be retrieved after teacher data is acquired are required. A constant sensor is required at a position where there is no singular point. The teacher data acquisition sensors need to be installed at predetermined intervals in a space where there is a singular point.

However, it is difficult for a service engineer who actually installs a sensor to install the sensor at an optimal position in a space if there is neither such knowledge nor a specified rule related to installation of sensors.

Although, as described above, when an air conditioner is to be optimally controlled using a sensor or when parameter distribution is to be outputted, for example, it is important to install the sensor at an optimal position, it has not been possible, in the related art, to find an optimal installation position for achieving target accuracy of prediction of a temperature, and to know an optimal number of sensors. Therefore, positions and a number of environment sensors have been determined depending on a sense of a service engineer, and there has been a case where it has been impossible to secure accuracy or a case where there has been a more cost than necessary due to excessive specifications.

With the sensor arrangement determination device 10, it is possible to take into consideration the influence of the air conditioner A on the temperature in the target space 80 to determine the arrangement candidate 31 for the constant sensor S1 and the arrangement candidate 32 for the teacher data acquisition sensor S2, making it possible to accurately predict the temperature in the target space 80.

Furthermore, with the sensor arrangement determination device 10, it is possible to determine the arrangement candidate 31 for the constant sensor S1 and the arrangement candidate 32 for the teacher data acquisition sensor S2 for achieving target accuracy of prediction of a temperature, making it possible to improve accuracy of air-conditioning control and accuracy of prediction of temperature distribution. Furthermore, with the sensor arrangement determination device 10, it is possible to reduce man-hours for examining installation positions for the constant sensor S1 and the teacher data acquisition sensor S2, and it is further possible to install the constant sensor S1 and the teacher data acquisition sensor S2 at optimal positions in the target space 80 without depending on the sense of a service engineer. Furthermore, with the sensor arrangement determination device 10, it is possible to acquire the air conditioner information 21 and the space information 22, making it possible to examine installation positions for the constant sensor S1 and the teacher data acquisition sensor S2 even without presence in an actual space. Furthermore, with the sensor arrangement determination device 10, it is possible to determine the arrangement candidate 31 for the constant sensor S1 and the arrangement candidate 32 for the teacher data acquisition sensor S2 to predict a temperature at another position where the teacher data acquisition sensor S2 is not arranged, making it possible to suppress a cost of the sensors.

(5-2)
In the sensor arrangement determination device 10 according to the present embodiment, the space information 22 includes information related to the design model 70 of the building.

With the sensor arrangement determination device 10, it is possible to acquire information related to the target space 80 from the design model 70 of the building. Accordingly, it is possible to appropriately determine the arrangement candidate 31 for the constant sensor S1 and the arrangement candidate 32 for the teacher data acquisition sensor S2 for each target space 80 in the building in which the sensors are to be installed.

(5-3)
In the sensor arrangement determination device 10 according to the present embodiment, a factor that influences the temperature in the target space 80 is the air conditioner A. The air conditioner information 21 includes information related to the influence on the temperature in accordance with the distance from the air conditioner A, which is determined in accordance with at least one or a combination of any of the model of the air conditioner A, the shape of the air conditioner (indoor unit) A, the capability of the air conditioner A, the air quantity of conditioned air supplied by the air conditioner A, and the system information of the air conditioner A.

With the sensor arrangement determination device 10, it is possible to take into consideration the influence of the air conditioner A on the temperature in the target space 80 to determine the arrangement candidate 31 for the constant sensor S1 and the arrangement candidate 32 for the teacher data acquisition sensor S2. Accordingly, it is possible to appropriately determine the arrangement candidate 31 for the constant sensor S1 and the arrangement candidate 32 for the teacher data acquisition sensor S2 in accordance with the air conditioner A installed in the target space 80.

(5-4)
In the sensor arrangement determination device 10 according to the present embodiment, the air conditioner information 21 further includes information related to the influence on the temperature in accordance with the distance from the air conditioner A, which is determined in accordance with at least one or a combination of any of the direction of wind, the blow-out temperature, the blow-out range, and the wind speed of the conditioned air supplied by the air conditioner A.

With the sensor arrangement determination device 10, it is possible to take into consideration the influence of the air-conditioned air supplied by the air conditioner A on the temperature in the target space 80 to determine the arrangement candidate 31 for the constant sensor S1 and the arrangement candidate 32 for the teacher data acquisition sensor S2.

(5-5)
In the sensor arrangement determination device 10 according to the present embodiment, the determination unit 30 calculates the floor area of the target space 80 from the space information 22, and determines the number 33 of the constant sensors S1 to be arranged in the target space 80 in accordance with the floor area of the target space 80.

With the sensor arrangement determination device 10, it is possible to take into consideration the size of the target space 80 to determine the installation number 33 of the constant sensors S1, making it possible to avoid arrangement a large number of sensors and to reduce a cost.

(5-6)
A sensor arrangement determination method using the sensor arrangement determination device 10 according to the present embodiment is a sensor arrangement determination method of determining arrangement of a sensor S that measures the temperature in the target space 80, the method including an acquisition step and a determination step. In the acquisition step, the air conditioner information 21 and the space information 22 are acquired. The air conditioner information 21 is information in which the air conditioner A and the influence of the distance from the air conditioner A are associated with each other. The space information 22 is information related to arrangement of the air conditioner A in the target space 80. In the determination step, the arrangement candidate 31 for the constant sensor S1 and the arrangement candidate 32 for the teacher data acquisition sensor S2 in the target space 80 are determined based on the air conditioner information 21 and the space information 22.

With the sensor arrangement determination method using the sensor arrangement determination device 10, it is possible to take into consideration the influence of the air conditioner A in the target space 80 on the temperature to determine the arrangement candidate 31 for the constant sensor S1 and the arrangement candidate 32 for the teacher data acquisition sensor S2, making it possible to accurately predict the temperature in the target space 80.

### (6) Modification Examples

### (6-1) Modification Example 1A

Although, in the sensor arrangement determination device 10 according to the present embodiment, the case where the factor that influences the temperature in the target space 80 is the air conditioner A has been described, the present invention is not limited to this example.

In the sensor arrangement determination device 10 illustrated in Fig. 1, a factor that influences a temperature in a target space 82 (see Fig. 8) may be the ventilation device B (see Fig. 8). Furthermore, in the sensor arrangement determination device 10 illustrated in Fig. 1, the factors that influence the temperature in the target space 82 may be the air conditioner A and the ventilation device B.

When the factor that influences the temperature in the target space 82 is the ventilation device B, the acquisition unit 20 acquires ventilation device information as the first information. The ventilation device information includes information related to an influence on the temperature in accordance with a distance from the ventilation device B, which is determined in accordance with at least one or a combination of any of a model of the ventilation device B, a shape of the ventilation device B, a ventilation quantity of the ventilation device B, an air quantity of air supplied by the ventilation device B, presence or absence of a total heat exchanger, heat exchange efficiency of the total heat exchanger, and presence or absence of a temperature adjustment function of the ventilation device B.

When the distance from the ventilation device B is short, the influence of the ventilation device B on the temperature in the target space 82 becomes large, and temperature change, for example, in the target space 82 becomes large due to the ventilation device B. For example, although, when the distance from the ventilation device B is approximately 3 m or shorter, the influence of the ventilation device B on the temperature in the target space 82 becomes large, the distance from the ventilation device B is not limited to this example.

Examples of the model of the ventilation device B include a ventilation fan, a total heat exchanger, and a humidity conditioning outdoor air processor. Examples of the shape of the ventilation device B include a propeller fan and a sirocco fan.

When the ventilation quantity of the ventilation device B is large, the influence of the ventilation device B on the temperature in the target space 82 becomes large. For example, although, when the ventilation quantity of the ventilation device B is approximately 300 m3/h or more, the influence of the ventilation device B on the temperature in the target space 82 becomes large, the ventilation quantity of the ventilation device B is not limited to this example.

When the air quantity of air supplied by the ventilation device B is large, the influence of the ventilation device B on the temperature in the target space 82 becomes large. For example, although, when the air quantity of air supplied by the ventilation device B is approximately 300 m3/h or more, the influence of the ventilation device B on the temperature in the target space 82 becomes large, the air quantity of air supplied by the ventilation device B is not limited to this example.

When the ventilation device B is a total heat exchanger, heat exchange takes place between air inside a room and air outside the room, and thus a temperature of air that is supplied from outside a room into inside the room approaches a temperature of the air inside the room. Therefore, the influence of the ventilation device B on the temperature in the target space 82 becomes small. Furthermore, when the heat exchange efficiency of the total heat exchanger is high, the influence of the ventilation device B on the temperature in the target space 82 becomes small.

Furthermore, when the ventilation device B has a temperature adjustment function, a magnitude of a degree of influence of the ventilation device B on the temperature in the target space 82 varies depending on content of operation of a temperature regulation function of the ventilation device B. When the ventilation device B having the temperature regulation function is operated to allow a temperature of air supplied to approach a temperature inside a room, the influence of the ventilation device B on the temperature in the target space 82 becomes small. Furthermore, when the ventilation device B having the temperature regulation function is operated to supply air having undergone temperature regulation to the target space 82, similar to the case of the air conditioner A, the influence of the ventilation device B on the temperature in the target space 82 becomes large.

Furthermore, associating characteristics of the ventilation device B and a reaching range of a blow-out airflow from the ventilation device B with each other and inputting ventilation device information to the sensor arrangement determination device 10 make it possible to acquire an influence range of the ventilation device B on the temperature in the target space.

In Modification Example 1A, it is possible to take into consideration the influences of the air conditioner A and the ventilation device B on the temperature in the target space 82 to determine the arrangement candidate 31 for the constant sensor S1 and the arrangement candidate 32 for the teacher data acquisition sensor S2 in the target space 82.

### (6-2) Modification Example 1B

In the sensor arrangement determination device 10 illustrated in Fig. 1, a factor that influences the temperature in the target space 82 may be a fixture C (see Fig. 8). Examples of the fixture include equipment and furniture used daily. Examples of the fixture further include partitions and shelves.

When a factor that influences the temperature in the target space 82 is the fixture C, the acquisition unit 20 acquires fixture information as the first information. The fixture information includes information related to an influence on the temperature in accordance with a distance from the fixture C, which is determined in accordance with a type of the fixture C. For example, since, when the fixture C arranged in the target space 82 is a partition, an airflow in the target space 82 changes around the partition, a measured value of a sensor is likely to be influenced by the partition.

When the distance from the fixture C is short, the influence of the fixture C on the temperature in the target space 82 becomes large, and temperature change, for example, in the target space 82 becomes large due to the fixture C. For example, although, when the fixture C is a partition and the distance from the fixture C is approximately 0.1 m or shorter, the influence of the fixture C on the temperature in the target space 82 becomes large, the distance from the fixture C is not limited to this example.

Furthermore, in the sensor arrangement determination device 10 illustrated in Fig. 1, a factor that influences the temperature in the target space 82 may be a fixture that generates heat (a heating body). Examples of the fixture that generates heat include a personal computer F (see Fig. 8) and a printer.

For example, when a fixture that generates heat is a desktop personal computer and a distance from the desktop personal computer having an amount of heat to be generated of 100 W is approximately 0.2 m or shorter, an influence of the desktop personal computer on the temperature in the target space 82 becomes large.

In Modification Example 1B, it is possible to take into consideration the influence of the fixture C on the temperature in the target space 82 to determine the arrangement candidate 31 for the constant sensor S1 and the arrangement candidate 32 for the teacher data acquisition sensor S2.

### (6-3) Modification Example 1C

In the sensor arrangement determination device 10 illustrated in Fig. 1, a factor that influences the temperature in the target space 82 may be the window D (see Fig. 8) provided in the target space 82. When a factor that influences the temperature in the target space 82 is the window D, the acquisition unit 20 acquires window information as the first information. The window information indicates an influence on the temperature in accordance with a distance from the window D, which is determined in accordance with at least one or a combination of any of a size, a heat transmission coefficient, and a solar radiation acquisition coefficient of the window D, and presence or absence of a shield.

When the distance from the window D is short, the influence of the window D on the temperature in the target space 82 becomes large, and temperature change, for example, in the target space 82 becomes large due to the window D. For example, although, when the distance from the window D is approximately 1.0 m or shorter, the influence of the window D on the temperature of the target space 82 becomes large, the distance from the window D is not limited to this example.

The heat transmission coefficient is a value indicating how easily heat is transferred when heat transfer occurs through the window D. It is possible to calculate the heat transmission coefficient using software. Furthermore, it is possible to measure the heat transmission coefficient using a heat flow sensor. When a temperature difference between outside and inside of the window D in the target space 82 is large, the heat transmission coefficient becomes large.

The solar radiation acquisition coefficient is an average ratio within a period of time between an "amount of solar radiation, which is able to be actually acquired inside a building" and an "amount of solar radiation, which is able to be acquired when it is assumed that there be no location shielded by a building". When the solar radiation acquisition coefficient is large, an amount of heat becomes large due to solar radiation.

Examples of the shield include a curtain, a blind, and a Japanese-style paper-fitted door, which shield solar radiation entering from the window D. When the window D is provided with a shield, a measured value of a sensor is less likely to be influenced by the window D.

Furthermore, when the window D is large, a measured value of a sensor is likely to be influenced around the window D.

In Modification Example 1C, it is possible to take into consideration the influence of the window D on the temperature in the target space 82 to determine the arrangement candidate 31 for the constant sensor S1 and the arrangement candidate 32 for the teacher data acquisition sensor S2.

### (6-4) Modification Example 1D

In the sensor arrangement determination device 10 illustrated in Fig. 1, a factor that influences the temperature in the target space 82 may be a door E (see Fig. 8) provided in the target space 82. When a factor that influences the environment in the target space 82 is the door E, the acquisition unit 20 acquires door information as the first information. The door information is the influence on the temperature in accordance with a distance from the door E, which is determined in accordance with at least one or a combination of any of a size, heat insulation performance, and an opening-and-closing frequency of the door E.

When the distance from the door E is short, the influence of the door E on the temperature in the target space 82 becomes large, and temperature change, for example, in the target space 82 becomes large due to the door E. For example, although, when the distance from the door E is approximately 2 m or shorter, the influence of the door E on the temperature in the target space 82 becomes large, the distance from the door E is not limited to the example.

When the door E is large, a measured value of a sensor is likely to be influenced around the door E.

Furthermore, when the heat insulation performance of the door E is high, heat exchange between inside and outside of the room is suppressed, and the measured value of the sensor in the target space 82 is less likely to be influenced by a temperature of outside air.

When the opening-and-closing frequency of the door E is high, heat exchange between inside and outside of the room is facilitated, and the measured value of the sensor is likely to be influenced.

In Modification Example 1D, it is possible to take into consideration the influence of the door E on the temperature in the target space 82 to determine the arrangement candidate 31 for the constant sensor S1 and a candidate, that is, the arrangement candidate 32 for the teacher data acquisition sensor S2.

### (6-5) Modification Example 1E

In the sensor arrangement determination device 10 illustrated in Fig. 1, the determination unit 30 may determine the number 33 of the constant sensors S1 to be arranged in the target space 82 in accordance with a number of factors that influence the temperature in the target space 82.

Fig. 8 is a diagram for explaining factors that influence the environment in the target space 82. As illustrated in Fig. 8, the target space 82 includes the air conditioners A1 to A4 (A3 and A4 are not illustrated), ventilation devices B1 and B2 (B2 is not illustrated), the fixtures C, the windows D, the door E, and the personal computers F.

As described in the present embodiment, the air conditioners A are factors that influence the temperature in the target space 82. The ventilation devices B, the fixtures C, the windows D, and the door E are factors that influence the temperature in the target space 82 as described in Modification Examples 1A to 1D. Furthermore, as described in Modification Example 1B, the personal computers F are heating bodies, and are factors that influence the temperature in the target space 82.

Furthermore, a skin load G illustrated in Fig. 8 is a thermal load that enters the room from outside through a skin such as a ceiling, a wall, or a floor in the target space 82, and is a factor that influences the temperature in the target space 82.

The acquisition unit 20 acquires the space information 22 (an architectural drawing, a piping drawing, a computer-aided designing (CAD) model, and a building information modeling (BIM) model, for example) of the target space 82 in which the constant sensor S1 and the teacher data acquisition sensor S2 are to be installed, installation positions of the air conditioners A (horizontal coordinates and heights of the indoor units, for example), installation positions of the ventilation devices B (horizontal coordinates and heights, for example), the air conditioner information 21 (models, shapes of the indoor units, capability, air quantities, and system information), ventilation device information (models, shapes, and air quantities, presence or absence of a total heat exchanger, heat exchange efficiency of the total heat exchanger, and presence or absence of a temperature adjustment function), positions of the fixtures C such as partitions, positions of heating bodies such as the personal computers F, positions of persons, the heat transmission coefficients of the windows D, a heat transmission coefficient of the door E, heat transmission coefficients of the walls, and the solar radiation acquisition coefficients of the windows D.

The determination unit 30 utilizes the air conditioner information 21, air-conditioning controller information, the ventilation device information, the space information 22 of the target space 82, the installation positions of the air conditioners A, the installation positions of the ventilation devices B, the positions of the fixtures C such as the partitions, the positions of the persons, the heat transmission coefficients of the windows D, the heat transmission coefficient of the door E, the heat transmission coefficients of the walls, and the solar radiation acquisition coefficients, for example, to calculate a position in the target space 82, at which environmental information (temperature information) that is necessary to "most efficiently control the space" and "correctly acquire real-time information in the space wholly" should be acquired.

In Modification Example 1E, it is possible to take into consideration a number of factors that influence the temperature in the target space 82 to determine the numerical value 33 of the constant sensor S1, making it possible to determine the optimal numerical value 33 of the constant sensor S1 regardless of whether or not the number of factors that influence the temperature in the target space 82 is large or small.

### (6-6) Modification Example 1F

Although, in the present embodiment, the case where an inappropriate arrangement position for the constant sensor S1 lies within the reaching range of the airflow from the air conditioner A has been described, the present invention is not limited to this example.

To efficiently control an air conditioner, sensing at a position where air conditioning is to be performed by each air conditioner is necessary.

However, for example, when air conditioning is controlled using a temperature detected by an environment sensor, and if the environment sensor is installed at a position of direct reception of a blow-out airflow from an air conditioner, it is difficult to grasp the environment at a position other than the position of reception of the airflow. Furthermore, when installed near a heating apparatus such as a personal computer, the environment sensor detects generated heat. In such a case, control to be performed is only directed to such a singular point, failing to achieve overall optimization.

However, when such environment sensors are to be installed based on an intuition and experience of a service engineer, there is a possibility that it be impossible to achieve installation at optimal positions, or, even when installation is possible, an excessive number of environment sensors may be installed, resulting in an increase in cost.

Furthermore, even when an environmental parameter is to be predicted, there is an identical or similar problem to that when an air conditioner is to be controlled. As an example implementation form, among environment sensors, a constant sensor is constantly installed, and an environment sensor that acquires teacher data is retrieved after the teacher data is acquired. In a possible form, a value of a constant sensor is used as an explanatory variable, and a temperature at a position where an environment sensor that acquires teacher data is installed is predicted in a real-time manner.

In this example, when the constant sensor is installed at a position where there is a singular point, there is a possibility that a parameter for the constant sensor, which serves as an explanatory variable, have an extra element for predicting a parameter for the environment sensor that acquires teacher data or there is insufficiency in element for prediction.

As an installation position of a constant sensor, it is necessary to perform installation at a position where there is no singular point. Furthermore, to acquire an environment in a whole space, it is necessary that an environment sensor that acquires teacher data acquire teacher data at each of a position where there is no singular point and a position where there is a singular point.

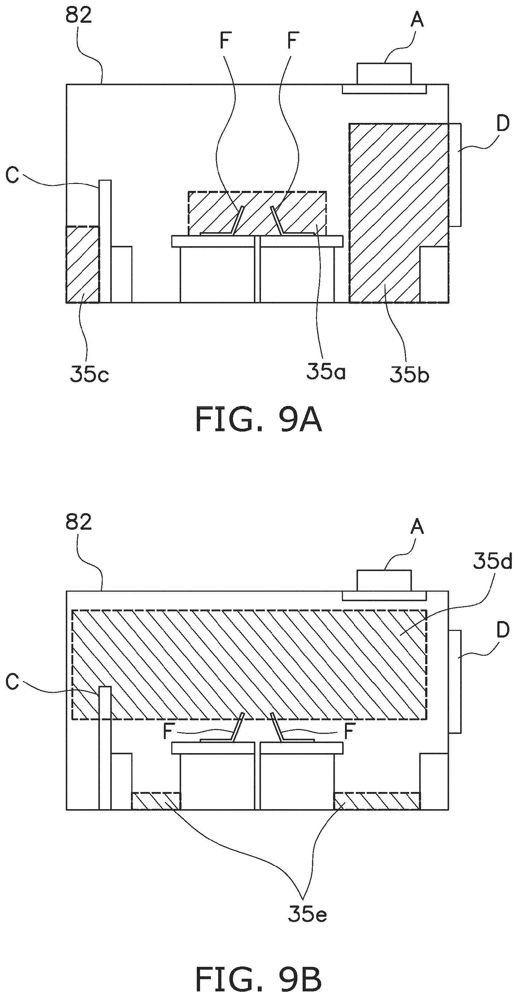

Figs. 9A and 9B are diagrams for explaining other example inappropriate arrangement positions for the constant sensor S1.

As illustrated in Fig. 9A, an inappropriate arrangement position for the constant sensor S1 is a position near a singular point. The singular point is a point where an environment (a temperature) is locally different from that in another surrounding area. Specific points are, for example, a position 35a near the personal computers F that are heating bodies, a position 35b where there is a strong influence of solar radiation entering from the window D, a position 35c behind the fixture C, when viewed from one of the blow-out ports of the air conditioner A, a position near the door E (see Fig. 8), and an area where many people are crowded, for example, in the target space 82.

For a heating body, a distance at which there is an influence on a temperature is stored in association with a type of and an amount of heat to be generated from the heating body, and a singular point in the target space 82 is derived from the space information 22 related to arrangement of the heating body in the target space 82.

For the window D, a peak thermal load is roughly calculated from the size, the heat transmission coefficient, and the solar radiation acquisition coefficient of the window D, and presence or absence of a shield, and a distance at which there is an influence on the temperature is calculated based on the roughly calculated thermal load to derive a singular point. At this time, those that it is possible to determine that there is a little influence on the temperature, such as a window on a side adjacent to another building and there is a little influence of solar radiation, may be excluded from the singular points.

Furthermore, for the door E, a distance at which there is an influence on the temperature is calculated by inputting the size, the heat transmission coefficient, and the opening-and-closing frequency of the door E to derive a singular point. At this time, those that it is possible to determine that there is a little influence on the temperature, such as a normally-closed door, may be excluded from the singular points.

Furthermore, as illustrated in Fig. 9B, inappropriate arrangement positions for the constant sensor S1 are installation-impossible positions. The installation-impossible positions include a position 35d and a floor surface 35e, for example, where it is impossible to physically install a sensor, such as a position in the air, in the target space 82.

Based on the space information 22, a range in which it is impossible to physically install a sensor, such as a position in the air, a position at which it is inconvenient for constant installation, such as a floor surface, and a position far away from a residential area, such as a ceiling, are acquired. Furthermore, for inappropriate arrangement positions (range) for the constant sensor S1, an installation prohibition range that is inputted by a user may be added.

In the sensor arrangement determination device 10 illustrated in Fig. 1, in each area divided based on a floor area of the target space 82 from a range excluding the inappropriate arrangement positions (range) for the constant sensor S1 in the target space 82, a position or a range, which is close to an intermediate position in the area, and at or in which installation of the constant sensor S1 is recommended, is determined.

For the teacher data acquisition sensor S2, a reaching range of an airflow, for which it is necessary to measure a temperature, and a singular point are determined based on the inappropriate arrangement positions (range) for the constant sensor S1, and then installation positions (range) for the teacher data acquisition sensors S2 are determined to allow the teacher data acquisition sensors S2 to wholly cover the target space 82 at predetermined distance intervals in the reaching range of the airflow and a range where there is no singular point. Distance intervals for the teacher data acquisition sensors S2 are determined based on target accuracy of prediction of a temperature.

Furthermore, for the teacher data acquisition sensors S2, a position in the air, which has been excluded as an inappropriate arrangement position (range) for the constant sensor S1, where, in this case, the teacher data acquisition sensors S2 are arranged using poles or tripods, for example, may be designated. When the teacher data acquisition sensors S2 are installed at positions in the air in the target space 82, it may be allowed to acquire temperature distribution in vertical directions in the target space 82.

In Modification Example 1F, positions that are likely to be singular points in the target spaces 80 and 82 are estimated from the space information 22 that the acquisition unit 20 acquires, and are excluded from arrangement candidates for the constant sensor S1. Accordingly, it is possible to take into consideration inappropriate arrangement positions for the constant sensor S1 to appropriately determine the arrangement candidate 31 for the constant sensor S1.

### (6-7) Modification Example 1G

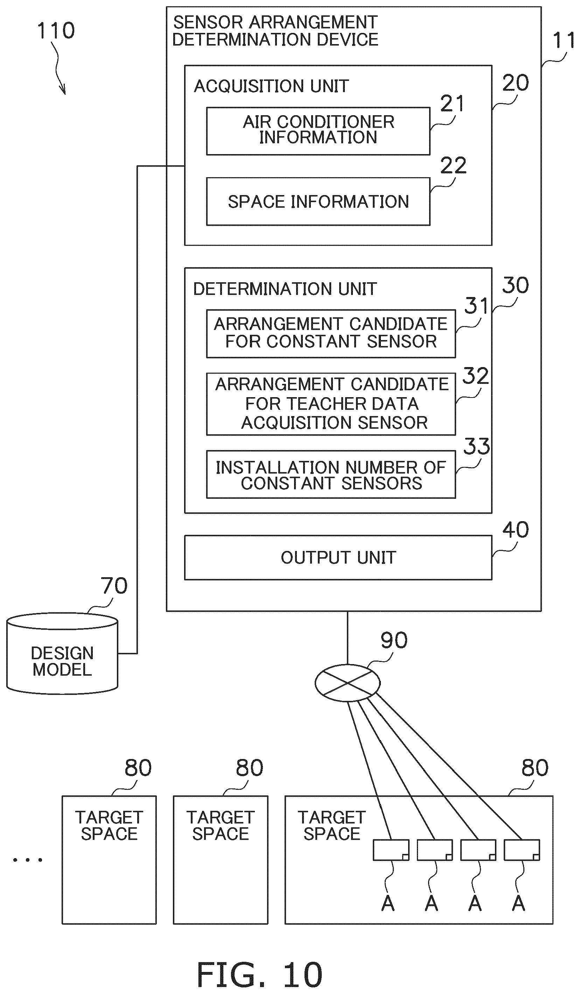

A functional block diagram of an air-conditioning control system 110 according to Modification example 1G is illustrated in Fig. 10. As illustrated in Fig. 10, the air-conditioning control system 110 includes the air conditioner A and a sensor arrangement determination device 11.

The sensor arrangement determination device 11 is identical or similar to the sensor arrangement determination device 10 in the air-conditioning control system 100 according to the present embodiment illustrated in Fig. 1 in that it includes the acquisition unit 20 and the determination unit 30. The air-conditioning control system 120 according to Modification Example 1G is different from the sensor arrangement determination device 10 illustrated in Fig. 1 in that the sensor arrangement determination device 11 further includes an output unit 40.

The output unit 40 outputs the arrangement candidate 31 for the constant sensor S1 in the target space 80 and the arrangement candidate 32 for the teacher data acquisition sensor S2 in the target space 80, which are determined by the determination unit 30.

The output unit 40 distinguishes a plurality of sensors having different purposes from each other, and outputs arrangement candidates for the sensors in the target space 80. In Modification Example 1G, the output unit 40 distinguishes the constant sensor S1 and the teacher data acquisition sensor S2 from each other, and outputs the arrangement candidate 31 for the constant sensor S1 in the target space 80 and the arrangement candidate 32 for the teacher data acquisition sensor S2 in the target space 80.

In Modification Example 1G, the target space may be, for example, the target space 82 including the air conditioners A, the ventilation devices B, and the like.

Fig. 11 illustrates an example of installation ranges for the constant sensor S1 and the teacher data acquisition sensors S2 in the target space 82 illustrated in Fig. 8.

As illustrated in Fig. 11, an installation candidate 31 for the constant sensor S1 is a position away from the air conditioners A1 to A4, the ventilation devices B1, B2, the fixtures C, the windows D, the door E, and the personal computers F (not illustrated). The constant sensor S1 is arranged, for example, at a position at a height ranging from 0.7 m to 0.8 m, which is a height from the floor surface to a top of a desk.

Furthermore, an arrangement candidate 32A for the teacher data acquisition sensor S2 is a position near a blow-out port of the ventilation device B1. Furthermore, an arrangement candidate 32B for the teacher data acquisition sensor S2 is a position near the fixtures C. Furthermore, an arrangement candidate 32C for the teacher data acquisition sensor S2 is a position near the air conditioner A1 and near some of the windows D. An arrangement candidate 32D for the teacher data acquisition sensor S2 is a position near the air conditioner A1. Furthermore, an arrangement candidate 32E for the teacher data acquisition sensor S2 is a position near the air conditioner A2. Furthermore, an arrangement candidate 32F for the teacher data acquisition sensor S2 is a position near the air conditioner A3. Furthermore, an arrangement candidate 32G for the teacher data acquisition sensor S2 is a position near the air conditioner A4. Furthermore, an arrangement candidate 32H for the teacher data acquisition sensor S2 is a position away from a blow-out port of the ventilation device B2. Furthermore, an arrangement candidate 32I for the teacher data acquisition sensor S2 is a position away from the blow-out port of the ventilation device B2. For example, the teacher data acquisition sensors S2 are arranged at positions each at a height ranging from 0.7 m to 0.8 m, which is the height from the floor surface to the top of the desk.

In the sensor arrangement determination device 11 according to Modification Example 1G, the model of the air conditioner A and an influence range (an influence range of airflow) on the temperature are associated with each other, or other factors that influence the temperature and influence ranges on the temperature are stored, information (air conditioner information, for example) related to the factors that influence the temperature is inputted, an influence range of a factor that influences an environment in a space is calculated, and installation guidance positions for the constant sensor S1 and the teacher data acquisition sensor S2, which are estimated based on the influence range of the factor, are displayed on a design drawing or a CAD model through an application running on an air-conditioning controller or a device such as a personal computer or a tablet. Accordingly, to correctly acquire an environment in a whole space or to control a target space, it is possible to facilitate installation of sensors at appropriate positions.

To control an air-conditioning device and a ventilation device and to actually measure and predict parameter distribution in a space, positions and a number of environment sensors to be installed are optimized, and control accuracy for the air conditioner and control accuracy for the ventilation device are improved, making it possible to correctly acquire the parameter distribution in the space. Furthermore, sensors more than necessary are not installed with respect to target accuracy of prediction of a temperature, and a cost for purchasing and a cost for managing sensors are reduced.

Furthermore, air conditioning may be controlled using actually-measured environmental information (actually-measured temperature information) acquired by the constant sensor S1 installed at a position calculated by the sensor arrangement determination device 11 according to Modification Example 1G, or may be controlled using predicted environmental information (predicted temperature information). Alternatively, the sensor arrangement determination device 11 may actually measure or predict and acquire environmental information (temperature information) in the target spaces 80 and 82.

In Modification Example 1G, optimal installation positions for the constant sensors S1 and the teacher data acquisition sensors S2 with respect to the target spaces 80 and 82 are automatically selected and presented to a service engineer. The output unit 40 is able to present installation recommendation positions (ranges) for the constant sensor S1 and the teacher data acquisition sensor S2 on a CAD model, a BIM model, and an architectural drawing of a building, or an application in a form of text, for example. Furthermore, it is possible to display the installation recommendation positions (ranges) for the constant sensor S1 and the teacher data acquisition sensor S2 on a user terminal, for example, via an application running on an air-conditioning controller or the user terminal, in a superimposed display manner on one from which an interior shape is visually recognizable, such as a CAD model, an architectural drawing, or a camera image, or in a form of text. Furthermore, the inappropriate arrangement position (range) for the constant sensor S1 may be presented as an installation prohibition range for the constant sensor S1.

In the sensor arrangement determination device 11 according to Modification Example 1G, the output unit 40 outputs the arrangement candidates 31 for the constant sensors S1 in the target spaces 80 and 82 and the arrangement candidates 32 for the teacher data acquisition sensors S2 in the target spaces 80 and 82, which are determined by the determination unit 30, allowing a user to easily acquire the arrangement candidates 31 for the constant sensors S1 and the arrangement candidates 32 for the teacher data acquisition sensors S2.

### (6-8) Modification Example 1H

In the sensor arrangement determination device 11 illustrated in Fig. 10, the determination unit 30 may determine, for each purpose of arranging sensors, either positions where there is an influence of a distance from a factor that influences the temperature in the target space 80 or positions where there is no influence of the distance from the factor that influences the temperature in the target space 80 as the arrangement candidate 31 for the constant sensor S1 or the arrangement candidate 32 for the teacher data acquisition sensor S2 in the target space 80.

Furthermore, in Modification Example 1H, the output unit 40 outputs, for each purpose of arranging sensors, the arrangement candidate 31 for the constant sensor S1 and the arrangement candidate 32 for the teacher data acquisition sensor S2 in the target space 80 or an inappropriate arrangement position for the constant sensor S1 in the target space 80.

In Modification Example 1H, the sensors having different purposes include the constant sensor S1 and the teacher data acquisition sensor S2. The constant sensor S1 is constantly installed in the target space 80. The teacher data acquisition sensor S2 is retrieved after data related to an environment in the space has been acquired.

In Modification Example 1H, for example, the output unit 40 is able to display the constant sensor S1 and the teacher data acquisition sensor S2 on an application running on the user terminal in a distinguishable manner.

The user may designate a position for the constant sensor S1, but, when the user designates a position for the constant sensor S1, a recommended position for the constant sensor S1 and the position designated by the user are displayed for comparison, and, further, it is indicated to the user that target accuracy of prediction of a temperature decreases.

Furthermore, the output unit 40 outputs a notification of a timing at which the teacher data acquisition sensor S2 is to be retrieved from the target space 80 or a notification of a timing at which the teacher data acquisition sensor S2 ends acquisition of data.

In Modification Example 1H, the target space may be, for example, the target space 82 including the air conditioners A, the ventilation devices B, and the like.

With the sensor arrangement determination device 11 according to Modification Example 1H, it is possible to acquire an inappropriate arrangement position for the constant sensor S1, making it possible to take into consideration the inappropriate arrangement position to determine the arrangement candidate 31 for the constant sensor S1 and the arrangement candidate 32 for the teacher data acquisition sensor S2. Furthermore, with the sensor arrangement determination device 11 according to Modification Example 1H, it is possible to acquire a timing at which the teacher data acquisition sensor S2 is to be retrieved or a timing at which the teacher data acquisition sensor S2 ends acquisition of data.

As a type of a sensor, a sensor for constant observation of a singular point may be installed at a singular point where there is a particularly strong influence on an environment, in addition to the constant sensor S1 and the teacher data acquisition sensor S2.

### (6-9) Modification Example 1I

In the sensor arrangement determination device 10 illustrated in Fig. 1, the determination unit 30 may calculate a volume of the target space 80 from the space information 22, and determine the number 33 of the constant sensors S1 to be arranged in the target space 80 in accordance with the volume of the target space 80. Furthermore, the target space may be, for example, the target space 82 including the air conditioners A, the ventilation devices B, and the like.

In Modification Example 1I, it is possible to take into consideration a size of the target space 80 to determine the numerical value 33 of the constant sensor S1, making it possible to avoid arrangement of a large number of sensors and to reduce a cost.

### (6-10) Modification Example 1J

In the sensor arrangement determination device 10 illustrated in Fig. 1, the determination unit 30 may determine the number 33 of the constant sensors S1 to be arranged in the target space in accordance with a number of refrigerant systems.

In Modification Example 1J, the air conditioner information (first information) 21 further includes information related to the influence on the temperature in accordance with the distance from each of the air conditioners A, which is determined in accordance with the number of the refrigerant systems.

The determination unit 30 may determine the number 33 of the constant sensors S1 to be arranged in the target space in accordance with a number of the air conditioners A. Furthermore, the air conditioner information 21 may further include information related to the influence on the temperature in accordance with the distance from each of the air conditioners A, which is determined in accordance with the number of the air conditioners A.

Fig. 12 is a diagram for explaining an installation number of the constant sensors S1 in a target space 84 in which multi-air conditioners for a building are installed. For example, a range in which an identical refrigerant pipe system is installed in the multi-air conditioners for the building is defined as one area, and one constant sensor is installed in the one area.

The air conditioners A1 to A4 belong to the identical refrigerant pipe system, and are installed in a first area 84a in the target space 84. The determination unit 30 determines that the number of the constant sensors S1 to be installed in the first area 84a in the target space 84 is "1".

Furthermore, the air conditioners A5 to A8 belong to an identical refrigerant pipe system, and are installed in a second area 84b in the target space 84. The determination unit 30 determines that the number of the constant sensors S1 to be arranged in the second area 84b in the target space 84 is "1".

Based on a shape and a size of the target space 84, which are the space information 22, the number of the air conditioners A, which is the air conditioner information 21, and target accuracy of prediction of a temperature, the target space 84 may be divided into several areas each within a range in which the air conditioners A belonging to the identical refrigerant pipe system are installed, and it may be assumed that each one of the constant sensors S1 be installed in each of the areas to determine the number of the constant sensors S1.

In Modification Example 1J, it is possible to take into consideration the information of the air conditioners A to determine the numerical value 33 of the constant sensor S1, making it possible to determine the number 33 of the constant sensor S1, which is suitable for the target space 84 in which the air conditioners A are installed.

### (6-11) Modification Example 1K

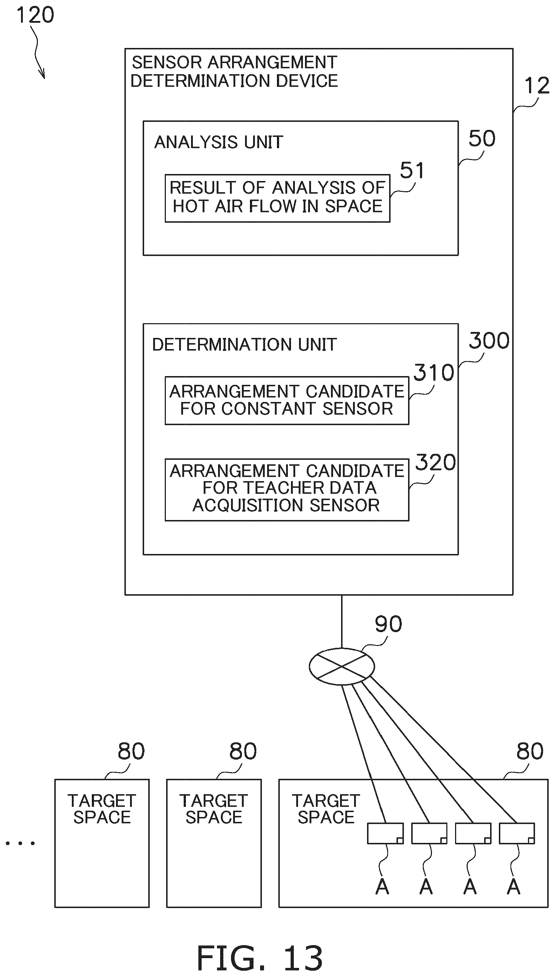

Fig. 13 is a functional block diagram of an air-conditioning control system 120 according to Modification Example 1K. As illustrated in Fig. 13, the air-conditioning control system 120 includes the air conditioner A and a sensor arrangement determination device 12.

The sensor arrangement determination device 12 according to Modification Example 1K includes an analysis unit 50 and a determination unit 300.

The analysis unit 50 performs, in relation to an influence, in the target space 80, of a factor that influences the temperature in the target space 80, analysis of a hot air flow in the target space 80.

The determination unit 300 determines an arrangement candidate 310 for the constant sensor S1 and an arrangement candidate 320 for the teacher data acquisition sensor S2 in the target space 80 based on a result 51 of the analysis of the hot air flow in the target space 80.

The target space may be, for example, the target space 82 including the air conditioners A, the ventilation devices B, and the like. An analysis model of the target space may be inputted as space information, and analysis of an airflow, such as computational fluid dynamics (CFD), may be performed to acquire an influence range of each of the air conditioners A, the ventilation devices B, and the like on the temperature in the target space.

With the sensor arrangement determination device 12 according to Modification Example 1K, it is possible to take into consideration the result 51 of the analysis of the hot air flow in the target space 80 to determine the arrangement candidate 310 for the constant sensor S1 and the arrangement candidate 320 for the teacher data acquisition sensor S2, making it possible to use the arrangement candidate 310 for the constant sensor S1 to be arranged and the arrangement candidate 320 for the teacher data acquisition sensor S2 to be arranged to accurately measure the temperature in the target space 80.

### (6-12) Modification Example 1L

Although, in the sensor arrangement determination device 10 according to the present embodiment, the case where the arrangement candidate 32 for the teacher data acquisition sensor S1 has been determined after the arrangement candidate 31 for the constant sensor S2 has been determined has been described, the order may be reversed, or determination may be performed simultaneously.

### (6-13) Modification Example 1M

Furthermore, in the sensor arrangement determination device 10 illustrated in Fig. 1, optimal positions and optimal numbers of the constant sensors S1 and the teacher data acquisition sensors S2 may be selected with respect to an existing space, or use of a simulation of arrangement positions for the constant sensors S1 and the teacher data acquisition sensors S2 with respect to a space in a design stage may be allowed.

### (6-14) Modification Example 1N

In the sensor arrangement determination device 10 illustrated in Fig. 1, it is possible to change positions of the constant sensor S1 and the teacher data acquisition sensor S2 in each season of spring, summer, autumn, and winter, making it also possible to designate installation positions for the sensors in accordance with each season. Furthermore, as a finer time span, positions of the constant sensor S1 and the teacher data acquisition sensor S2 may be changed in a time series of morning, afternoon, and evening.

### (6-15) Modification Example 1O

Although, in the present embodiment and Modification Examples, the cases where the sensors are temperature sensors have been described, example sensors may include a humidity sensor and a carbon dioxide concentration sensor, for example.

### (6-16)

While embodiments of the present invention have been described above, it will be understood that various changes in form and detail may be made therein within the scope of the appended claims. The above embodiments, when not within the scope of the appended claims, which define the invention, are present for illustration purposes only.

### REFERENCE SIGNS LIST

100, 110, 120 Air-conditioning control system
10, 11, 12 Sensor arrangement determination device
20 Acquisition unit
21 Air conditioner information (first information)
22 Space information (second information)
30, 300 Determination unit
31, 310 Arrangement candidate for constant sensor
32 (32a to 32d, 32A to 32I), 320 Arrangement candidate for teacher data acquisition sensor
33 Installation number of constant sensors
40 Output unit
50 Analysis unit
51 Result of analysis of hot air flow in space
A (A1 to A8) Air conditioner
B (B1, B2) Ventilation device
S1 Constant sensor
S2 Teacher data acquisition sensor

### CITATION LIST

### PATENT LITERATURE

PTL 1: Japanese Patent No. 3548114

## Claims

1. A sensor arrangement determination device (10, 11) configured to determine the arrangement of a sensor (S1, S2) which is configured to measure an environment in a space,
the space being a room,
the environment being a temperature,
the sensor arrangement determination device comprising:
an acquisition unit (20) configured to acquire second information (22) that is information related to arrangement of the factor in the space,
the factor being an air conditioning indoor unit (A);
a determination unit (30) configured to determine an arrangement candidate (31, 32) for the sensor in the space based on the second information; and
an output unit (40) configured to output the arrangement candidate determined by the determination unit (30),
**characterized in that**:
the acquisition unit is configured to further acquire first information (21) that is information in which the factor that influences the environment and an influence of a distance from the factor on the environment are associated with each other,
the influence being determined in accordance with at least one or a combination of any of a model of the indoor unit, capability of the indoor unit, and system information of the indoor unit; and
the determination unit is configured to determine the arrangement candidate further based on the first information,
wherein:
the sensor includes a plurality of sensors arranged in the space for different purposes, the sensors including
a first sensor (S1) that is a temperature sensor for acquiring data related to the temperature in the space, and constantly installed in the space, and
a second sensor (S2) that is a temperature sensor for acquiring teacher data related to the temperature in the space, and installed in the space and to be retrieved after the teacher data has been acquired;
the determination unit is configured to determine a position where there is not a singular point as the arrangement candidate for the first sensor, and determine the arrangement candidate for the second sensor,
the singular point being a point where the environment is locally different from that in another surrounding area; and
the output unit (40) is configured to distinguish the first sensor and the second sensor from each other, and output the arrangement candidates for the first and second sensors or an inappropriate arrangement position for the first sensor in the space,
the inappropriate arrangement position being a position near the singular point.

2. The sensor arrangement determination device (10, 11) according to claim 1, wherein
the second information includes information related to at least one of a design model, an analysis model, an architectural drawing, and an instrumentation drawing for a building.

3. The sensor arrangement determination device (10, 11) according to claim 1 or 2, wherein
the first information further includes information related to an influence on the environment in accordance with a distance from the indoor unit,
the influence being determined in accordance with at least one or a combination of any of a shape of the indoor unit and an air quantity of conditioned air supplied by the indoor unit.

4. The sensor arrangement determination device according to claim 3, wherein
the first information further includes information related to an influence on the environment in accordance with a distance from the indoor unit,
the influence being determined in accordance with at least one or a combination of any of a direction of wind, a blow-out temperature, a blow-out range, and a wind speed of the conditioned air supplied by the indoor unit.

5. The sensor arrangement determination device (10, 11) according to any one of claims 1 to 4, wherein
the first information further includes information related to an influence on the environment in accordance with a distance from the indoor unit,
the influence being determined in accordance with a number of the indoor units or a number of refrigerant systems, and
the determination unit (30) is configured to determine a number of the sensors (S1, S2) to be arranged in the space in accordance with the number of the indoor units or the number of the refrigerant systems.

6. The sensor arrangement determination device (10, 11) according to any one of claims 1 to 4, wherein
the determination unit (30) is configured to determine a number of the sensors (S1, S2) to be arranged in the space in accordance with a number of the factors.

7. The sensor arrangement determination device (10, 11) according to any one of claims 1 to 6, wherein
the output unit (40) is configured to output a notification of a timing at which the second sensor (S2) is to be retrieved from the space or a notification of a timing at which the second sensor (S2) ends acquisition of the data.

8. A sensor arrangement determination method of determining arrangement of a sensor that measures an environment in a space,
the space being a room,
the environment being a temperature,
the sensor arrangement determination method being executed by a computer, and comprising:
an acquisition step (S11) of acquiring second information that is information related to arrangement of the factor in the space,
the factor being an air conditioning indoor unit;
a determination step (S15, S16) of determining an arrangement candidate for the sensor in the space based on the first information and the second information; and
an output step of outputting the arrangement candidate determined by the determination step,
**characterized in that**:
in the acquisition step, first information is further acquired that is information in which a factor that influences the environment and an influence of a distance from the factor on the environment are associated with each other,
the influence being determined in accordance with at least one or a combination of any of a model of the indoor unit, capability of the indoor unit, and system information of the indoor unit; and
in the determination step, the arrangement candidate is determined further based on the first information,
wherein:
the sensor includes a plurality of sensors arranged in the space for different purposes, the sensors including
a first sensor (S1) that is a temperature sensor for acquiring data related to the temperature in the space, and constantly installed in the space, and
a second sensor (S2) that is a temperature sensor for acquiring teacher data related to the temperature in the space, and installed in the space and to be retrieved after the teacher data has been acquired;
in the determining step, a position where there is not a singular point is determined as the arrangement candidate for the first sensor, and the arrangement candidate for the second sensor is determined,
the singular point being a point where the environment is locally different from that in another surrounding area; and
in the output step, the first sensor and the second sensor are distinguished from each other, and the arrangement candidates for the first and second sensors or an inappropriate arrangement position for the first sensor in the space is outputted,
the inappropriate arrangement position being a position near the singular point.

## Patentansprüche

1. Sensoranordnungs-Bestimmungsvorrichtung (10, 11), die konfiguriert ist, die Anordnung eines Sensors (S1, S2) zu bestimmen, der konfiguriert ist, eine Umgebung in einem Raum zu messen,
wobei der Raum ein Zimmer ist,
wobei die Umgebung eine Temperatur ist,
wobei die Sensoranordnungs-Bestimmungsvorrichtung aufweist:
eine Erfassungseinheit (20), die konfiguriert ist, zweite Informationen (22) zu erfassen, die Informationen sind, die sich auf die Anordnung des Faktors in dem Raum beziehen,
wobei der Faktor eine Klimatisierungs-Inneneinheit (A) ist;
eine Bestimmungseinheit (30), die konfiguriert ist, basierend auf den zweiten Informationen einen Anordnungskandidaten (31, 32) für den Sensor in dem Raum zu bestimmen; und
eine Ausgabeeinheit (40), die konfiguriert ist, den von der Bestimmungseinheit (30) bestimmten Anordnungskandidaten auszugeben,
**dadurch gekennzeichnet, dass**:
die Erfassungseinheit konfiguriert ist, ferner erste Informationen (21) zu erfassen, die Informationen sind, in denen der Faktor, der die Umgebung beeinflusst, und ein Einfluss eines Abstands von dem Faktor auf die Umgebung miteinander assoziiert sind,
wobei der Einfluss gemäß mindestens einem oder einer Kombination von irgendeinem eines Modells der Inneneinheit, einer Leistungsfähigkeit der Inneneinheit und Systeminformationen der Inneneinheit bestimmt wird; und
die Bestimmungseinheit konfiguriert ist, den Anordnungskandidaten ferner basierend auf den ersten Informationen zu bestimmen,
wobei:
der Sensor mehrere Sensoren aufweist, die in dem Raum für unterschiedliche Zwecke angeordnet sind, wobei die Sensoren aufweisen:
einen ersten Sensor (S1), der ein Temperatursensor zum Erfassen von Daten in Bezug auf die Temperatur im Raum ist und ständig im Raum installiert ist, und
einen zweiten Sensor (S2), der ein Temperatursensor zum Erfassen von Lehrerdaten in Bezug auf die Temperatur im Raum ist und im Raum installiert ist und herausgenommen werden soll, nachdem die Lehrerdaten erfasst worden sind;
die Bestimmungseinheit konfiguriert ist, eine Position, an der kein singulärer Punkt vorhanden ist, als Anordnungskandidaten für den ersten Sensor zu bestimmen und den Anordnungskandidaten für den zweiten Sensor zu bestimmen,
wobei der singuläre Punkt ein Punkt ist, an dem sich die Umgebung lokal von der in einem anderen umgebenden Bereich unterscheidet; und
die Ausgabeeinheit (40) konfiguriert ist, den ersten Sensor und den zweiten Sensor voneinander zu unterscheiden und die Anordnungskandidaten für den ersten und den zweiten Sensor oder eine ungeeignete Anordnungsposition für den ersten Sensor im Raum auszugeben,
wobei die ungeeignete Anordnungsposition eine Position in der Nähe des singulären Punkts ist.

2. Sensoranordnungs-Bestimmungsvorrichtung (10, 11) nach Anspruch 1, wobei die zweiten Informationen Informationen aufweisen, die sich auf mindestens eines von einem Entwurfsmodell, einem Analysemodell, einer Bauzeichnung und einer Installationszeichnung für ein Gebäude beziehen.

3. Sensoranordnungs-Bestimmungsvorrichtung (10, 11) nach Anspruch 1 oder 2, wobei
die ersten Informationen ferner Informationen enthalten, die sich auf einen Einfluss auf die Umgebung gemäß einem Abstand zur Inneneinheit beziehen,
wobei der Einfluss gemäß mindestens einem oder einer Kombination von irgendeinem einer Form der Inneneinheit und einer Luftmenge klimatisierter Luft bestimmt wird, die von der Inneneinheit zugeführt wird.

4. Sensoranordnungs-Bestimmungsvorrichtung nach Anspruch 3, wobei die ersten Informationen ferner Informationen enthalten, die sich auf einen Einfluss auf die Umgebung gemäß einem Abstand von der Inneneinheit beziehen,
wobei der Einfluss gemäß mindestens einem oder einer Kombination von irgendeinem einer Windrichtung, Ausblastemperatur, Ausblasreichweite und einer Windgeschwindigkeit der von der Inneneinheit zugeführten klimatisierten Luft bestimmt wird.

5. Sensoranordnungs-Bestimmungsvorrichtung (10, 11) nach einem der Ansprüche 1 bis 4, wobei
die ersten Informationen ferner Informationen aufweisen, die sich auf einen Einfluss auf die Umgebung gemäß einem Abstand zur Inneneinheit beziehen,
wobei der Einfluss gemäß einer Anzahl der Inneneinheiten oder einer Anzahl von Kältemittelsystemen bestimmt wird, und
die Bestimmungseinheit (30) konfiguriert ist, eine Anzahl der Sensoren (S1, S2), die in dem Raum angeordnet werden sollen, gemäß der Anzahl der Inneneinheiten oder der Anzahl der Kältemittelsysteme zu bestimmen.

6. Sensoranordnungs-Bestimmungsvorrichtung (10, 11) nach einem der Ansprüche 1 bis 4, wobei
die Bestimmungseinheit (30) konfiguriert ist, eine Anzahl der Sensoren (S1, S2), die in dem Raum angeordnet werden sollen, entsprechend einer Anzahl der Faktoren zu bestimmen.

7. Sensoranordnungs-Bestimmungsvorrichtung (10, 11) nach einem der Ansprüche 1 bis 6, wobei
die Ausgabeeinheit (40) konfiguriert ist, eine Benachrichtigung über einen Zeitpunkt, zu dem der zweite Sensor (S2) aus dem Raum herausgenommen werden soll, oder eine Benachrichtigung über einen Zeitpunkt auszugeben, zu dem der zweite Sensor (S2) die Erfassung der Daten beendet.

8. Sensoranordnungs-Bestimmungsverfahren zum Bestimmen einer Anordnung eines Sensors, der eine Umgebung in einem Raum misst,
wobei der Raum ein Zimmer ist,
wobei die Umgebung eine Temperatur ist,
wobei das Sensoranordnungs-Bestimmungsverfahren von einem Computer ausgeführt wird und aufweist:
einen Erfassungsschritt (S11) zum Erfassen von zweiten Informationen, die Informationen sind, die sich auf die Anordnung des Faktors in dem Raum beziehen,
wobei der Faktor eine Klimatisierungs-Inneneinheit ist;
einen Bestimmungsschritt (S15, S16) zum Bestimmen eines Anordnungskandidaten für den Sensor in dem Raum basierend auf den ersten Informationen und den zweiten Informationen; und
einen Ausgabeschritt zum Ausgeben des durch den Bestimmungsschritt bestimmten Anordnungskandidaten,
**dadurch gekennzeichnet, dass**:
im Erfassungsschritt ferner erste Informationen erfasst werden, die Informationen sind, in denen ein Faktor, der die Umgebung beeinflusst, und ein Einfluss eines Abstands von dem Faktor auf die Umgebung miteinander assoziiert sind,
wobei der Einfluss gemäß mindestens einem oder einer Kombination von irgendeinem eines Modells der Inneneinheit, einer Leistungsfähigkeit der Inneneinheit und Systeminformationen der Inneneinheit bestimmt wird; und
im Bestimmungsschritt der Anordnungskandidat ferner basierend auf der ersten Informationen bestimmt wird,
wobei:
der Sensor mehrere Sensoren aufweist, die in dem Raum für unterschiedliche Zwecke angeordnet sind, wobei die Sensoren aufweisen:
einen ersten Sensor (S1), der ein Temperatursensor zum Erfassen von Daten in Bezug auf die Temperatur im Raum ist und ständig im Raum installiert ist, und
einen zweiten Sensor (S2), der ein Temperatursensor zum Erfassen von Lehrerdaten in Bezug auf die Temperatur im Raum ist und im Raum installiert ist und herausgenommen werden soll, nachdem die Lehrerdaten erfasst worden sind;
im Bestimmungsschritt eine Position, an der kein singulärer Punkt vorhanden ist, als Anordnungskandidat für den ersten Sensor bestimmt wird, und der Anordnungskandidat für den zweiten Sensor bestimmt wird,
wobei der singuläre Punkt ein Punkt ist, an dem sich die Umgebung lokal von der in einem anderen umgebenden Bereich unterscheidet; und
im Ausgabeschritt der erste Sensor und der zweite Sensor voneinander unterschieden werden, und die Anordnungskandidaten für den ersten und den zweiten Sensor oder eine ungeeignete Anordnungsposition für den ersten Sensor im Raum ausgegeben werden,
wobei die ungeeignete Anordnungsposition eine Position in der Nähe des singulären Punkts ist.

## Revendications

1. Dispositif de détermination de positionnement d'un capteur (10, 11), prévu pour déterminer le positionnement d'un capteur (S1, S2) destiné à mesurer un environnement dans un espace,
ledit espace étant une pièce,
ledit environnement étant une température,
ledit dispositif de détermination de positionnement de capteur comprenant :
une unité d'acquisition (20) prévue pour acquérir des informations secondaires (22), lesquelles sont des informations relatives au positionnement du facteur dans l'espace,
ledit facteur étant une unité intérieure de climatisation (A) ;
une unité de détermination (30) prévue pour déterminer un emplacement candidat (31, 32) pour le capteur dans l'espace sur la base des informations secondaires ; et
une unité de sortie (40) prévue pour transmettre l'emplacement candidat déterminé par l'unité de détermination (30),
**caractérisé en ce que** :
l'unité d'acquisition est prévue pour acquérir en outre des informations primaires (21), en l'occurrence des informations où le facteur influençant l'environnement et l'influence de la distance du facteur sur l'environnement sont associées,
l'influence étant déterminée en fonction d'un modèle de l'unité intérieure et/ou des capacités de l'unité intérieure et/ou d'informations système de l'unité intérieure ; et **en ce que**
l'unité de détermination est prévue pour déterminer en outre la configuration candidate sur la base des informations primaires,
où :
le capteur comprend une pluralité de capteurs disposés dans l'espace à des fins différentes, lesdits capteurs comprenant
un premier capteur (S1), qui est un capteur de température destiné à acquérir des données relatives à la température dans l'espace, et qui est installé de manière permanente dans l'espace, et
un deuxième capteur (S2), qui est un capteur de température destiné à acquérir des données de référence relatives à la température dans l'espace, et qui est installé dans l'espace et doit être retiré une fois les données de référence acquises ;
l'unité de détermination est prévue pour déterminer une position où aucun point singulier n'est présenté comme emplacement candidat pour le premier capteur, et pour déterminer l'emplacement candidat pour le deuxième capteur,
le point singulier étant un point où l'environnement est localement différent de celui d'une autre zone environnante ; et
l'unité de sortie (40) est prévue pour distinguer le premier capteur du deuxième capteur, et pour transmettre les emplacements candidats pour le premier et le deuxième capteur ou une position d'installation inappropriée pour le premier capteur dans l'espace,
la position d'installation inappropriée étant une position proche du point singulier.

2. Dispositif de détermination de positionnement de capteur (10, 11) selon la revendication 1, où
les informations secondaires comprennent des informations relatives à un modèle de conception et/ou un modèle d'analyse et/ou un plan d'architecture et/ou un plan d'instrumentation d'un bâtiment.

3. Dispositif de détermination de positionnement de capteur (10, 11) selon la revendication 1 ou la revendication 2, où
les informations primaires comprennent en outre des informations relatives à une influence sur l'environnement en fonction d'une distance par rapport à l'unité intérieure,
l'influence étant déterminée en fonction de la forme de l'unité intérieure et/ou des capacités de l'unité intérieure et/ou du débit d'air conditionné refoulé par l'unité intérieure.

4. Dispositif de détermination de positionnement de capteur selon la revendication 3, où
les informations primaires comprennent en outre des informations relatives à une influence sur l'environnement en fonction d'une distance par rapport à l'unité intérieure,
l'influence étant déterminée en fonction de la direction du vent et/ou de la température de soufflage et/ou de la portée de soufflage et/ou de la vitesse de vent de l'air conditionné refoulé par l'unité intérieure.

5. Dispositif de détermination de positionnement de capteur (10, 11) selon l'une des revendications 1 à 4, où
les informations primaires comprennent en outre des informations relatives à une influence sur l'environnement en fonction d'une distance par rapport à l'unité intérieure,
l'influence étant déterminée en fonction d'un nombre d'unités intérieures ou d'un nombre de systèmes de réfrigération, et
l'unité de détermination (30) est prévue pour déterminer un nombre de capteurs (S1, S2) à disposer dans l'espace en fonction du nombre d'unités intérieures ou du nombre de systèmes de réfrigération.

6. Dispositif de détermination de positionnement de capteur (10, 11) selon l'une des revendications 1 à 4, où
l'unité de détermination (30) est prévue pour déterminer un nombre de capteurs (S1, S2) à disposer dans l'espace en fonction d'un nombre de facteurs.

7. Dispositif de détermination de positionnement de capteur (10, 11) selon l'une des revendications 1 à 6, où
l'unité de sortie (40) est prévue pour émettre une notification indiquant le moment où le deuxième capteur (S2) doit être retiré de l'espace ou une notification indiquant le moment où le deuxième capteur (S2) termine l'acquisition des données.

8. Procédé de détermination de positionnement d'un capteur mesurant un environnement dans un espace,
ledit espace étant une pièce,
ledit environnement étant une température,
ledit procédé de détermination de positionnement d'un capteur étant exécuté par un ordinateur, et comprenant :
une étape d'acquisition (S11) consistant à acquérir des informations secondaires,
lesquelles sont des informations relatives au positionnement du facteur dans l'espace,
ledit facteur étant une unité intérieure de climatisation ;
une étape de détermination (S15, S16) consistant à déterminer un emplacement candidat pour le capteur dans l'espace sur la base des informations primaires et des informations secondaires ; et
une étape de sortie consistant à transmettre l'emplacement candidat déterminé lors de l'étape de détermination,
**caractérisé en ce que** :
lors de l'étape d'acquisition, des informations primaires sont également acquises, en l'occurrence des informations où un facteur influençant l'environnement et une influence de la distance du facteur sur l'environnement sont associées,
l'influence étant déterminée en fonction d'un modèle de l'unité intérieure et/ou des capacités de l'unité intérieure et/ou d'informations système de l'unité intérieure ; et,
lors de l'étape de détermination, la configuration candidate est en outre déterminée sur la base des informations primaires,
où :
le capteur comprend une pluralité de capteurs disposés dans l'espace à des fins différentes, lesdits capteurs comprenant
un premier capteur (S1), qui est un capteur de température destiné à acquérir des données relatives à la température dans l'espace, et qui est installé de manière permanente dans l'espace, et
un deuxième capteur (S2), qui est un capteur de température destiné à acquérir des données de référence relatives à la température dans l'espace, et qui est installé dans l'espace et doit être retiré une fois les données de référence acquises ;
lors de l'étape de détermination, une position où aucun point singulier n'est présenté est déterminée comme emplacement candidat pour le premier capteur, et l'emplacement candidat est déterminé pour le deuxième capteur,
le point singulier étant un point où l'environnement est localement différent de celui d'une autre zone environnante ; et,
lors de l'étape de sortie, le premier capteur et le deuxième capteur sont distingués l'un de l'autre, et les emplacements candidats pour le premier et le deuxième capteur ou une position d'installation inappropriée pour le premier capteur dans l'espace sont transmis,
la position d'installation inappropriée étant une position proche du point singulier.
